# EUROPEAN PATENT APPLICATION

(11) **EP 4 015 040 A1**
(43) Date of publication of application: **22.06.2022**
(21) Application number: 20383104.5
(22) Date of filing: 16.12.2020
(51) Int. Cl.: A61P 7/00, A61K 31/7088, C12N 15/115

(54) **METHOD FOR TREATING BLOOD DISEASES**

(71) Applicant: Fundación para la Formación e Investigación Sanitarias de la Región de Murcia, 30120 Murcia (ES); Universidad de Murcia, 30003 Murcia (ES); Consorcio Centro de Investigación Biomédica en Red M.P., 28029 Madrid (ES)
(72) Inventor: CAYUELA FUENTES, María Luisa, 30120 Murcia (ES); ALCARAZ PÉREZ, Francisca, 30120 Murcia (ES); GARCÍA CASTILLO, Jesús, 30120 Murcia (ES); MULERO MÉNDEZ, Victoriano Francisco, 30003 Murcia (ES); GARCÍA MORENO, Diana, 30003 Murcia (ES); MARTÍNEZ BALSALOBRE, Elena, 30003 Murcia (ES); PÉREZ OLIVA, Ana Belén, 30003 Murcia (ES)
(74) Representative: Hoffmann Eitle

(57) **Abstract**

The present invention refers to a method for treating neutropenia and/or for the activation of myelopoiesis. For that purpose, affinity reagents, preferably aptamers, have been developed in the present invention which specifically bind the telomerase RNA component (TERC) DNA-binding-site.

SEQUENCE LISTING

<110>   Fundación para la formación e investigación sanitarias de la Región
de Murcia
        Universidad de Murcia

<120>  METHOD FOR TREATING BLOOD DISEASES

<130>  905803

<160>  26

<170>  PatentIn version 3.5

<210>  1
<211>  18
<212>  DNA
<213>  Artificial sequence

<220>
<223>  Artificial sequence



<220>
<221>  N_region
<222>  (2)..(2)
<223>  A or C

<220>
<221>  misc_feature
<222>  (2)..(6)
<223>  n is a, c, g, or t

<220>
<221>  N_region
<222>  (3)..(3)
<223>  A or C

<220>
<221>  N_region
<222>  (4)..(4)
<223>  T or A

<220>
<221>  N_region
<222>  (5)..(5)
<223>  G or T

<220>
<221>  N_region
<222>  (6)..(6)
<223>  G or C

<220>
<221>  N_region
<222>  (9)..(9)
<223>  A or C

<220>
<221>  misc_feature
<222>  (9)..(9)
<223>  n is a, c, g, or t

<220>
<221> N_region
<222> (12)..(12)
<223> A or C

<220>
<221> misc_feature
<222> (12)..(12)
<223> n is a, c, g, or t

<220>
<221> N_region
<222> (14)..(14)
<223> C or T

<220>
<221> misc_feature
<222> (14)..(18)
<223> n is a, c, g, or t

<220>
<221> N_region
<222> (15)..(15)
<223> C or T

<220>
<221> N_region
<222> (16)..(16)
<223> C or T

<220>
<221> N_region
<222> (17)..(17)
<223> T or C

<220>
<221> N_region
<222> (18)..(18)
<223> C or A

<400> 1
annnnnccnc cncnnnnn 18


<210> 2
<211> 84
<212> RNA
<213> Artificial sequence

<220>
<223> Artificial sequence

<400> 2
cccgccugga ggccgcgguc ggcccggggc uucuccggag gcacccacug ccaccgcgaa 60

gaguugggcu cugucagccg cggg 84


<210> 3
<211> 15
<212> RNA
<213> Artificial sequence

<220>
<223>  Artificial sequence

<400>  3
aagaguuggg cucug                                                        15


<210>  4
<211>  22
<212>  DNA
<213>  Artificial sequence

<220>
<223>  Artificial sequence

<400>  4
cacaggtttg gctgtttgta cg                                                22


<210>  5
<211>  22
<212>  DNA
<213>  Artificial sequence

<220>
<223>  Artificial sequence

<400>  5
gaaggtttgc ggacctcaaa ca                                                22


<210>  6
<211>  18
<212>  DNA
<213>  Artificial sequence

<220>
<223>  Artificial sequence

<400>  6
caagagttcg gctctgct                                                     18


<210>  7
<211>  22
<212>  DNA
<213>  Artificial sequence

<220>
<223>  Artificial sequence

<400>  7
cacaggtttg gctgtttgta cg                                                22


<210>  8
<211>  20
<212>  DNA
<213>  Artificial sequence

<220>
<223>  Artificial sequence

<400>  8
ggtctcacag gtttggctgt                                                   20


<210>  9
<211>  20
<212>  DNA
<213>  Artificial sequence

<220>
<223>  Artificial sequence

<400>  9
tgcaggatca gtgtttgagg                                                   20


<210>  10
<211>  20
<212>  DNA
<213>  Artificial sequence

<220>
<223>  Artificial sequence

<400>  10
ggagctctgc gcacccaaca                                                   20


<210>  11
<211>  20
<212>  DNA
<213>  Artificial sequence

<220>
<223>  Artificial sequence

<400>  11
ggcagggctc cagcagcttc                                                   20


<210>  12
<211>  20
<212>  DNA
<213>  Artificial sequence

<220>
<223>  Artificial sequence

<400>  12
tgttaccctc acaacgtcca                                                   20


<210>  13
<211>  20
<212>  DNA
<213>  Artificial sequence

<220>
<223>  Artificial sequence

<400>  13
gcagaaggtc aagcaggaac                                                   20

<210>  14
<211>  20
<212>  DNA
<213>  Artificial sequence

<220>
<223>  Artificial sequence

<400>  14
cgttgggtgt cccccggtct                                                    20


<210>  15
<211>  20
<212>  DNA
<213>  Artificial sequence

<220>
<223>  Artificial sequence

<400>  15
acgaggctcg gctctggacg                                                    20


<210>  16
<211>  20
<212>  DNA
<213>  Artificial sequence

<220>
<223>  Artificial sequence

<400>  16
acagaaatgc cccttcactg                                                    20


<210>  17
<211>  20
<212>  DNA
<213>  Artificial sequence

<220>
<223>  Artificial sequence

<400>  17
gcctcttctc aaaacggttc                                                    20


<210>  18
<211>  23
<212>  DNA
<213>  Artificial sequence

<220>
<223>  Artificial sequence

<400>  18
ggaagctctt catcaccttc agg                                                23


<210>  19
<211>  79

<212>  RNA
<213>  Artificial sequence

<220>
<223>  Artificial sequence

<400>  19
cacagguuug gcuguuugua cgcggacagu uugacugccg ccaagaguuc ggcucugcug      60

cacauucggc aggucugug                                                   79


<210>  20
<211>  17
<212>  RNA
<213>  Artificial sequence

<220>
<223>  Artificial sequence

<400>  20
caagaguucg gcucugc                                                     17


<210>  21
<211>  17
<212>  RNA
<213>  Artificial sequence

<220>
<223>  Artificial sequence

<400>  21
caagaauucg gcucugc                                                     17


<210>  22
<211>  44
<212>  RNA
<213>  Artificial sequence

<220>
<223>  Artificial sequence

<400>  22
cacagguuug gcuguuugua cgcugcacau ucggcagguc ugug                       44


<210>  23
<211>  44
<212>  RNA
<213>  Artificial sequence

<220>
<223>  Artificial sequence

<400>  23
cacagguuug gcuguuugua cgcuucucau ucggcagguc ugug                       44


<210>  24
<211>  46
<212>  RNA

<213>  Artificial sequence

<220>
<223>  Artificial sequence

<400>  24
uggcgaaggu uugcggaccu caaacacuga uccugcaaau acgcca                    46


<210>  25
<211>  15
<212>  RNA
<213>  Artificial sequence

<220>
<223>  Artificial sequence

<400>  25
aagaauuggg cucug                                                      15


<210>  26
<211>  46
<212>  RNA
<213>  Artificial sequence

<220>
<223>  Artificial sequence

<400>  26
ccccgcgcgc ggcgcgauuc ccugagcugu gggacgugca cccagg                    46

SEQUENCE LISTING

<110>  Fundación para la Formación e Investigación Sanitarias de la Región
De Murcia
       Universidad de Murcia

<120>  METHOD FOR TREATING BLOOD DISEASES

<130>  905803

<160>  28

<170>  PatentIn version 3.5

<210>  1
<211>  18
<212>  DNA
<213>  Artificial sequence

<220>
<223>  Artificial sequence



<220>
<221>  N_region
<222>  (2)..(2)
<223>  A or C

<220>
<221>  misc_feature
<222>  (2)..(6)
<223>  n is a, c, g, or t

<220>
<221>  N_region
<222>  (3)..(3)
<223>  A or C

<220>
<221>  N_region
<222>  (4)..(4)
<223>  T or A

<220>
<221>  N_region
<222>  (5)..(5)
<223>  G or T

<220>
<221>  N_region
<222>  (6)..(6)
<223>  G or C

<220>
<221>  N_region
<222>  (9)..(9)
<223>  A or C

<220>
<221>  misc_feature
<222>  (9)..(9)
<223>  n is a, c, g, or t

<220>
<221> N_region
<222> (12)..(12)
<223> A or C

<220>
<221> misc_feature
<222> (12)..(12)
<223> n is a, c, g, or t

<220>
<221> N_region
<222> (14)..(14)
<223> C or T

<220>
<221> misc_feature
<222> (14)..(18)
<223> n is a, c, g, or t

<220>
<221> N_region
<222> (15)..(15)
<223> C or T

<220>
<221> N_region
<222> (16)..(16)
<223> C or T

<220>
<221> N_region
<222> (17)..(17)
<223> T or C

<220>
<221> N_region
<222> (18)..(18)
<223> C or A

<400> 1
annnnnccnc cncnnnnn 18


<210> 2
<211> 84
<212> RNA
<213> Artificial sequence

<220>
<223> Artificial sequence

<400> 2
cccgccugga ggccgcgguc ggcccggggc uucuccggag gcacccacug ccaccgcgaa 60

gaguugggcu cugucagccg cggg 84


<210> 3
<211> 15
<212> RNA
<213> Artificial sequence

<220>
<223>  Artificial sequence

<400>  3
aagaguuggg cucug                                                        15


<210>  4
<211>  22
<212>  DNA
<213>  Artificial sequence

<220>
<223>  Artificial sequence

<400>  4
cacaggtttg gctgtttgta cg                                                22


<210>  5
<211>  22
<212>  DNA
<213>  Artificial sequence

<220>
<223>  Artificial sequence

<400>  5
gaaggtttgc ggacctcaaa ca                                                22


<210>  6
<211>  18
<212>  DNA
<213>  Artificial sequence

<220>
<223>  Artificial sequence

<400>  6
caagagttcg gctctgct                                                     18


<210>  7
<211>  22
<212>  DNA
<213>  Artificial sequence

<220>
<223>  Artificial sequence

<400>  7
cacaggtttg gctgtttgta cg                                                22


<210>  8
<211>  20
<212>  DNA
<213>  Artificial sequence

<220>
<223>  Artificial sequence

<400>  8
ggtctcacag gtttggctgt                                                   20


<210>  9
<211>  20
<212>  DNA
<213>  Artificial sequence

<220>
<223>  Artificial sequence

<400>  9
tgcaggatca gtgtttgagg                                                   20


<210>  10
<211>  20
<212>  DNA
<213>  Artificial sequence

<220>
<223>  Artificial sequence

<400>  10
ggagctctgc gcacccaaca                                                   20


<210>  11
<211>  20
<212>  DNA
<213>  Artificial sequence

<220>
<223>  Artificial sequence

<400>  11
ggcagggctc cagcagcttc                                                   20


<210>  12
<211>  20
<212>  DNA
<213>  Artificial sequence

<220>
<223>  Artificial sequence

<400>  12
tgttaccctc acaacgtcca                                                   20


<210>  13
<211>  20
<212>  DNA
<213>  Artificial sequence

<220>
<223>  Artificial sequence

<400>  13
gcagaaggtc aagcaggaac                                                   20

<210>  14
<211>  20
<212>  DNA
<213>  Artificial sequence

<220>
<223>  Artificial sequence

<400>  14
cgttgggtgt cccccggtct                                                    20


<210>  15
<211>  20
<212>  DNA
<213>  Artificial sequence

<220>
<223>  Artificial sequence

<400>  15
acgaggctcg gctctggacg                                                    20


<210>  16
<211>  20
<212>  DNA
<213>  Artificial sequence

<220>
<223>  Artificial sequence

<400>  16
acagaaatgc cccttcactg                                                    20


<210>  17
<211>  20
<212>  DNA
<213>  Artificial sequence

<220>
<223>  Artificial sequence

<400>  17
gcctcttctc aaaacggttc                                                    20


<210>  18
<211>  23
<212>  DNA
<213>  Artificial sequence

<220>
<223>  Artificial sequence

<400>  18
ggaagctctt catcaccttc agg                                                23


<210>  19
<211>  79

<212>  RNA
<213>  Artificial sequence

<220>
<223>  Artificial sequence

<400>  19
cacagguuug gcuguuugua cgcggacagu uugacugccg ccaagaguuc ggcucugcug      60

cacauucggc aggucugug                                                   79


<210>  20
<211>  17
<212>  RNA
<213>  Artificial sequence

<220>
<223>  Artificial sequence

<400>  20
caagaguucg gcucugc                                                     17


<210>  21
<211>  17
<212>  RNA
<213>  Artificial sequence

<220>
<223>  Artificial sequence

<400>  21
caagaauucg gcucugc                                                     17


<210>  22
<211>  44
<212>  RNA
<213>  Artificial sequence

<220>
<223>  Artificial sequence

<400>  22
cacagguuug gcuguuugua cgcugcacau ucggcagguc ugug                       44


<210>  23
<211>  44
<212>  RNA
<213>  Artificial sequence

<220>
<223>  Artificial sequence

<400>  23
cacagguuug gcuguuugua cgcuucucau ucggcagguc ugug                       44


<210>  24
<211>  46
<212>  RNA

<213>  Artificial sequence

<220>
<223>  Artificial sequence

<400>  24
uggcgaaggu uugcggaccu caaacacuga uccugcaaau acgcca                    46


<210>  25
<211>  15
<212>  RNA
<213>  Artificial sequence

<220>
<223>  Artificial sequence

<400>  25
aagaauuggg cucug                                                      15


<210>  26
<211>  46
<212>  RNA
<213>  Artificial sequence

<220>
<223>  Artificial sequence

<400>  26
ccccgcgcgc ggcgcgauuc ccugagcugu gggacgugca cccagg                    46

<210>  27
<211>  451
<212>  RNA
<213>  Artificial sequence

<220>
<223>  Artificial sequence

<400>  27
   gguuugcgga gggugggccu gggaggggug guggccauuu uuugucuaac ccuaacugag      60
   aagggcguag gcgccgugcu uuugcucccc gcgcgcuguu uuucucgcug acuuucagcg     120
   gcgcgaaaag ccucggccug ccgccuucca ccguucauuc uagagcaaac aaaaaauguc     180
   agcugcuggc ccguucgccc cucccgggga ccugcggcgg gucgccugcc cagcccccga     240
   accccgccug gaggccgcgg ucggcccggg gcuucuccgg aggcacccac ugccaccgcg     300
   aagaguuggg cucugucagc cgcgggucuc ucgggggcga gggcgagguu caggccuuuc     360
   aggccgcagg aagaggaacg gagcgagucc ccgcgcgcgg cgcgauuccc ugagcugugg     420
   gacgugcacc caggacucgg cucacacaug c                                    451


<210>  28
<211>  317
<212>  RNA
<213>  Artificial sequence

<220>
<223>  Artificial sequence

<400>  28
gcagaguacu uucucuaacc cuaacgcuuc uuugcuuccu guacggcuug uaugcuguuu      60

ugucuuaagc uuucagcaua uuacaugaag cuuuccgcgg uuucgauacu uuaaaaaagc     120

uauagaguac ucuggcgcuc ggucucacag guuuggcugu uuguacgcgg acaguuugac     180

ugccgccaag aguucggcuc ugcugcacau ucggcagguc uguggacugc acaacacuga     240

gcagauuaac uugagcaugg cgaagguuug cggaccucaa acacugaucc ugcaaauacg     300

ccaccgcuca gacaugc                                                    317

## Description

### FIELD OF THE INVENTION

The present invention refers to the medical field. Particularly, it refers to a method for treating neutropenia and/or for the activation of myelopoiesis. For that purpose, affinity reagents, preferably aptamers, have been developed in the present invention which specifically bind the telomerase RNA component (TERC) DNA-binding-site.

### PRIOR ART

Telomerase is an RNA-dependent DNA polymerase that synthesizes telomeric repeats at the end of eukaryotic chromosomes to prevents telomeric shortening. This enzyme consists mainly of a protein component (TERT) and an RNA component (TERC). TERC has a very complex structure characterized by the presence of three conserved structural domains: the pseudoknot/core domain, which contains the template sequence and is essential for telomerase activity, the conserved regions 4 and 5 (CR4/CR5), which is critical for TERT association, and the ScaRNA domain, which contains H/ACA and CR7 domains that are responsible for interacting with other telomerase-associated proteins. Each domain is divided into subdomains, specifically the CR4/CR5 domain consists of P5, P6 and P6.1 regions connected as a three-way junction.

Telomerase mutations are associated with premature ageing, while telomerase reactivation in adult cells is associated with cancer. However, numerous studies have shown that both components of telomerase complex are involved in several cell signalling pathways without an apparent involvement of its well-established function in telomere maintenance.

Neutropenia is a dangerous and potentially fatal disease characterized by a lower than normal number of neutrophils in the circulation, either due to reduced production, high elimination or a storage problem. Neutropenia can be caused by multiple factors, such as chemotherapy (the most common cause), drugs, infections, autoimmune diseases, bone marrow disorders, chronic idiopathic neutropenia or genetic disorder as happen in dyskeratosis congenita (DC). DC is an inherited sickness associated with mutations in telomerase components or in telomere-stabilizing components. The principal cause of premature mortality is bone marrow failure, associated with the development of aplastic anaemias and myelodysplastic syndromes. The incidence of these hematopoietic phenotypes is higher in patients harbouring mutations that affect TERC compared to those patients with other mutations, and this observation cannot be explained only by telomere shortening. It is tempting to speculate, therefore, that these hematopoietic syndromes are related with the role of TERC in myelopoiesis.

The currently available treatment for neutropenia is the administration of granulocyte colony stimulating factor (G-CSF). G-CSF has been approved by the Food and Drug Administration (FDA) for the treatment of congenital and acquired neutropenia and for the mobilization of peripheral hematopoietic progenitor cells for stem cell transplantation. In addition, G-CSF therapy has been used in DC patients, being relatively effective in children and young adults with DC. However, G-CSF treatment has some side effects (dyspnea, chest pain, nausea, hypoxemia, diaphoresis, anaphylaxis, syncope flushing and splenomegaly), but the biggest disadvantage is its high cost.

So, there is an unmet medical need of finding cost-effective therapies for the treatment of neutropenia (or diseases derived thereof) and/or for the activation of myelopoiesis. The present invention is focused on solving this problem and it is herein provided a new therapy aimed at treating neutropenia and/or activating myelopoiesis. Preferably, this new therapy is based on aptamers which are synthetic single-stranded DNA or RNA sequences that adopt unique three-dimensional structures that allow them to recognize a specific target with high affinity.

### DESCRIPTION OF THE INVENTION

Such as it is explained above, the present invention refers to a method for treating neutropenia and/or for the activation of myelopoiesis.

Particularly, several affinity reagents, particularly aptamers, have been developed in the present invention which specifically bind the TERC DNA-binding-site.

Kindly note that TERC is one of the main components of telomerase complex and contains the template for telomere elongation. TERC has a non-canonical function in myelopoiesis acting as a transcriptional factor that binds to a consensus DNA binding sequence and attracts RNA polymerase II (RNA Pol II), facilitating myeloid gene expression. TERC regulates the expression of the master myelopoiesis genes csf3b and spilb in zebrafish, and CSF1, CSF2, CSF3 and SPI1 in humans. Importantly, the CR4/CR5 domain of TERC plays a fundamental role in myelopoiesis, since a mutation of this domain found in dyskeratosis congenita (DC) patients decreases its affinity for RNA Pol II, impairing its myelopoietic activity. Consequently, the inventors of the present invention have developed and assayed different aptamers to be used as high affinity agents for disease treatment. Particularly, several aptamers based on the CR4/CR5 sequence were designed to investigate if these small molecules were able to increase myelopoiesis. After an exhaustive screening of different aptamer candidates, the results provided in the present invention indicate that two specific aptamers were able to increase the number of neutrophils, without affecting the number of erythrocytes in zebrafish. Mechanistically, the aptamers functioned as full TERC, that is, they increased the expression of master myeloid genes independently of endogenous TERC, and they bind to TERC-binding sequences of DNA found in the regulatory regions of these target genes and to RNA Pol II, enforcing their transcription. Importantly, aptamers harbouring CR4/CR5 mutations found in DC patients failed to perform all these functions. Preclinical zebrafish models of DC (TERC deficiency) and poikiloderma with neutropenia (usb1 deficiency) diseases demonstrated the therapeutic potential of the developed aptamers to treat neutropenia. Finally, the corresponding human aptamers also increased myeloid gene expression promoting myelopoiesis in human and in human induced pluripotent stem cells. Therefore, two potential therapeutic agents for DC and other neutropenic patients have been developed in the present invention.

So, the first embodiment of the present invention refers to affinity reagents specifically binding the TERC DNA-binding-site consisting of the sequence: A/C/G A/C/G A/C/T T/A/C G/T/A/G G/C/T/A C/G C A/C C C/A A/C C/A/T C/T/A C/T/A/G C/T/A/G T/C/A C/A/G), preferably consisting of SEQ ID NO: 1 (A A/C A/C T/A G/T G/C C C A/C C C A/C C C/T C/T C/T T/C C/A) for use as a medicament.

In a preferred embodiment, the affinity reagents specifically bind the TERC DNA-binding-site consisting of SEQ ID NO: 1 at the regulatory regions of the genes CSF1, CSF2, CSF3 and SPI1.

In a preferred embodiment, the affinity reagents are used in the treatment of neutropenia and/or the activation of myelopoiesis.

In a preferred embodiment, the affinity reagents are used in the treatment of dyskeratosis congenita, poikiloderma with neutropenia, Charcot Marie tooth disease, non-immune chronic idiopathic neutropenia of adult, severe congenital neutropenia SCN8, SCN1, SCN3 or SCNX, anaemia x-linked with neutropenia and/or platelet abnormalities, cyclic neutropenia, Barth syndrome (BTHS), Shwachman-Diamond syndrome, Chédiak-Higashi syndrome, WHIM syndrome, and glycogen storage disease type Ib, hyper IgM syndrome, Hermansky-Pudlak syndrome (HPS), Griscelli syndrome (GS), P14 deficiency, Cohen syndrome, mobilization of hematopoietic stem cells or chemotherapy-induced neutropenia.

In a preferred embodiment, the affinity reagents are aptamers consisting of SEQ ID NO: 2: CCCGCCUGGAGGCCGCGGUCGGCCCGGGGCUUCUCCGGAGGCACCCACUGCC ACCGCGAAGAGUUGGGCUCUGUCAGCCGCGGG (hereinafter CR4CR5) or SEQ ID NO: 3: AAGAGUUGGGCUCUG (hereinafter AA).

Alternatively, the present invention refers to a method for treating neutropenia and/or for the activation of myelopoiesis, particularly for treating dyskeratosis congenita, poikiloderma with neutropenia, Charcot Marie tooth disease, non-immune chronic idiopathic neutropenia of adult, severe congenital neutropenia SCN8, SCN1, SCN3 or SCNX, anaemia x-linked with neutropenia and/or platelet abnormalities, cyclic neutropenia, Barth syndrome (BTHS), Shwachman-Diamond syndrome, Chédiak-Higashi syndrome, WHIM syndrome, and glycogen storage disease type Ib, hyper IgM syndrome, Hermansky-Pudlak syndrome (HPS), Griscelli syndrome (GS), P14 deficiency, Cohen syndrome, mobilization of hematopoietic stem cells or chemotherapy-induced neutropenia; which comprises the administration of a pharmaceutically effective amount of an affinity reagent specifically binding the TERC DNA-binding-site consisting of SEQ ID NO: 1, preferably the aptamers of SEQ ID NO: 2 or SEQ ID NO: 3.

The second embodiment of the present invention refers to an aptamer consisting of SEQ ID NO: 2 or SEQ ID NO: 3. Moreover, the present invention also covers possible modifications of these aptamers, for instance: PEGylation or similar, locked nucleic acids, 2' sugar ring substitution, phosphonothioate replacement, Spiegelmer, slow off-rate-modified aptamers, conjugation to nanomaterials, conjugation to human serum albumin or to Cholesterol and Other Lipid Moieties or IgG conjugation or artificial nucleotides substitution

The third embodiment of the present invention refers to a pharmaceutical composition comprising any of the above-mentioned aptamers and, optionally, pharmaceutically acceptable excipients or carriers.

The fourth embodiment of the present invention refers to an *in vitro* method for screening affinity reagent candidates for the treatment of neutropenia and/or for the activation of myelopoiesis, which comprises determining whether the affinity reagent candidate binds the TERC DNA-binding-site consisting of SEQ ID NO: 1, wherein if it is determined that the affinity reagent candidate is able to bind the TERC DNA-binding-site consisting of SEQ ID NO: 1, the affinity reagent can be used for the treatment of neutropenia and/or for the activation of myelopoiesis.

In a preferred embodiment, the present invention refers to an *in vitro* method for screening affinity reagent candidates for the treatment of neutropenia and/or for the activation of myelopoiesis which comprises determining whether the affinity reagent candidate binds the DNA-binding-site consisting of SEQ ID NO: 1 at the regulatory regions of the genes gcsfb and spi-1 like of zebrafish or CSF1, CSF2, CSF3 and SPI1 human genes, wherein if it is determined that the affinity reagent candidate is able to bind the TERC DNA-binding-site consisting of SEQ ID NO: 1 at the regulatory regions of the genes gcsfb and spi-1 like of zebrafish or CSF1, CSF2, CSF3 and SPI1 human genes, the affinity reagent can be used for the treatment of neutropenia and/or for the activation of myelopoiesis.

In a preferred embodiment, the present invention refers to an *in vitro* method for screening affinity reagent candidates for the treatment of: dyskeratosis congenita, poikiloderma with neutropenia, Charcot Marie tooth disease, non-immune chronic idiopathic neutropenia of adult, severe congenital neutropenia SCN8, SCN1, SCN3 or SNCX, x-linked severe congenital neutropenia, anaemia x-linked with neutropenia and/or platelet abnormalities, cyclic neutropenia, Barth syndrome (BTHS), Shwachman-Diamond syndrome, Chédiak-Higashi syndrome, WHIM syndrome, and glycogen storage disease type Ib, hyper IgM syndrome, Hermansky-Pudlak syndrome (HPS), Griscelli syndrome (GS), P14 deficiency, Cohen syndrome, mobilization of stem cells or chemotherapy-induced neutropenia, which comprises determining whether the affinity reagent candidate binds the TERC DNA-binding-site consisting of SEQ ID NO: 1 at the regulatory regions of gcsfb and spi-1 like genes of zebrafish or CSF1, CSF2, CSF3 and SPI1 human genes, wherein if it is determined that the affinity reagent candidate is able to bind the TERC DNA-binding-site consisting of SEQ ID NO: 1 at the regulatory regions of gcsfb and spi-1 like genes of zebrafish or CSF1, CSF2, CSF3 and SPI1 human genes, the affinity reagent can be used for the treatment of: dyskeratosis congenita, poikiloderma with neutropenia, Charcot Marie tooth disease, non-immune chronic idiopathic neutropenia of adult, severe congenital neutropenia SCN8, SCN1 or SCN3, x-linked severe congenital neutropenia, anaemia x-linked with neutropenia and/or platelet abnormalities, cyclic neutropenia, Barth syndrome (BTHS), Shwachman-Diamond syndrome, Chédiak-Higashi syndrome, WHIM syndrome, and glycogen storage disease type Ib, hyper IgM syndrome, Hermansky-Pudlak syndrome (HPS), Griscelli syndrome (GS), P14 deficiency, Cohen syndrome, mobilization of stem cells or chemotherapy-induced neutropenia.

In a preferred embodiment, the present invention refers to an *in vitro* method for screening aptamer candidates.

The fifth embodiment of the present invention refers to an *in vitro* method for generating affinity reagents specifically binding the TERC DNA-binding-site consisting of SEQ ID NO: 1 which comprises the use of the CR4/CR5 domain of TERC. In a preferred embodiment, the present invention refers to an *in vitro* method for generating aptamers specifically binding the TERC DNA-binding-site consisting of SEQ ID NO: 1 which comprises the use of the CR4/CR5 domain of TERC.

The sixth embodiment of the present invention refers to the *in vitro* use of CR4/CR5 domain of TERC for the generation of affinity reagents able to bind the TERC DNA-binding-site consisting of SEQ ID NO: 1. In a preferred embodiment, the present invention refers to the *in vitro* use of CR4/CR5 domain of TERC for the generation of aptamers able to bind the TERC DNA-binding-site consisting of SEQ ID NO: 1.

For the purpose of the present invention the following terms are defined:
- By "neutropenia" it is understood a specific disease characterized by a lower than normal number of neutrophils in the circulation. Absolute neutrophil count (ANC) is a measure of the number of neutrophil granulocytes present in the blood: mild neutropenia (ANC less than 1.5 × 10⁹/l), moderate neutropenia (ANC between 0.5 and 1.5 × 10⁹/l), and severe neutropenia (ANC less than 0.5 × 10⁹/l) either due to reduced production, high elimination or a storage problem. So, it is understood that the term "neutropenia" is clear because instructions are available from the patent document and from the Common General Knowledge which allows the skilled person to recognise which conditions fall within this definition.
- By "affinity reagent" it is understood a compound that binds specific substances, such as proteins or nucleic acids. They term may include, for instance, aptamers, antibodies or other small molecules that specifically bind to a larger target molecule in order to identify, track, capture, or influence its activity.
- The term "comprising" means including, but it is not limited to, whatever follows the word "comprising". Thus, use of the term "comprising" indicates that the listed elements are required or mandatory, but that other elements are optional and may or may not be present.

- By "consisting of' means including, and it is limited to, whatever follows the phrase "consisting of'. Thus, the phrase "consisting of' indicates that the listed elements are required or mandatory, and that no other elements may be present.
- By "therapeutically effective dose or amount" is intended an amount that, when administered as described herein, brings about a positive therapeutic response in a subject suffering from neutropenia. The exact amount required will vary from subject to subject, depending on the age, and general condition of the subject, the severity of the condition being treated, mode of administration, and the like. An appropriate "effective" amount in any individual case may be determined by one of ordinary skill in the art using routine experimentation, based upon the information provided herein.

### Brief description of the figures

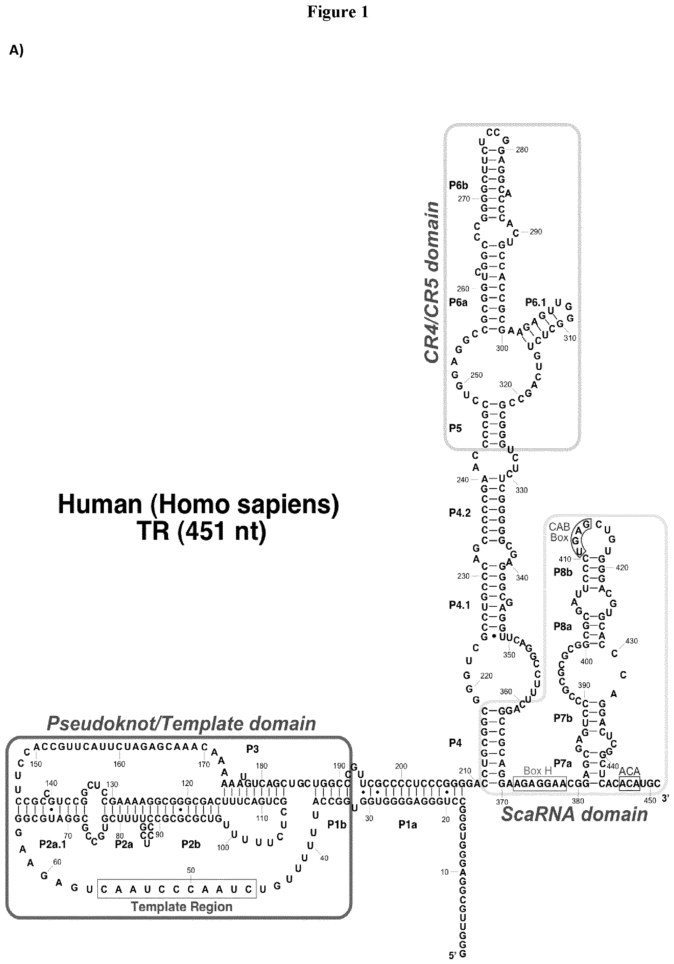

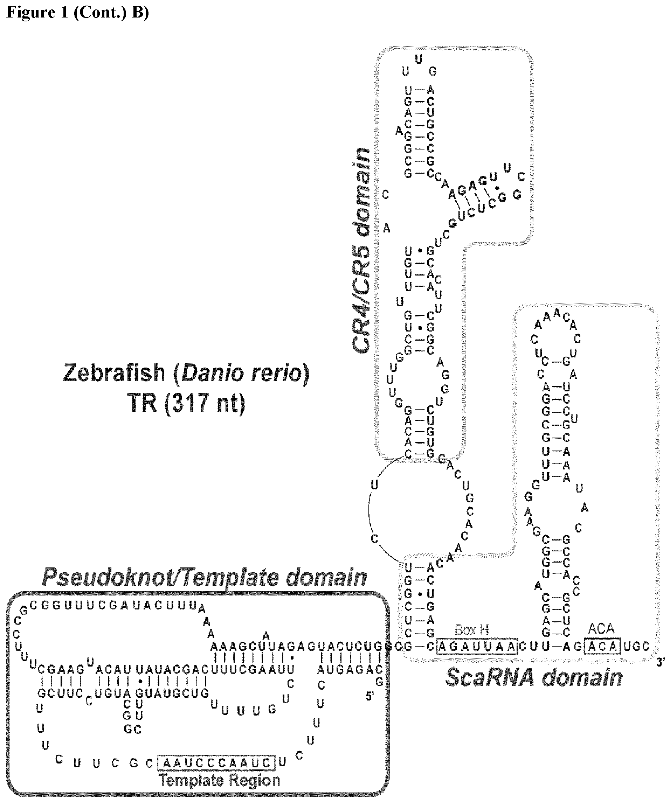

**Figure 1****. Mapping of TERC mutations. (a)** Human TERC. **(b)** Zebrafish terc. Mutations are color-coded according to the disease in which they were first identified. Mutations responsible for myelodysplastic syndrome and aplastic anaemia of CR4/CR5 domain are marked in pink and purple, respectively.

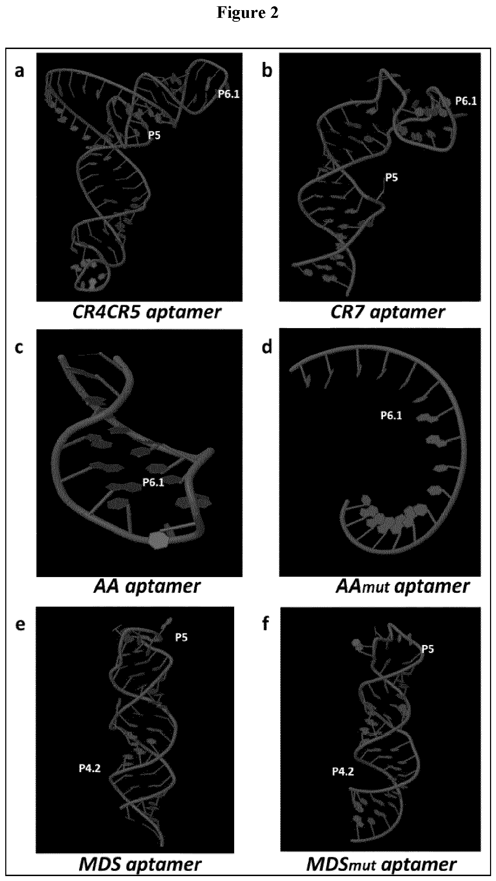

**Figure 2****. 3D zebrafish aptamers structure.** This is a prediction and modelling of tertiary RNA structure using the RNAComposer system (http://rnacomposer.cs.put.poznan.pl/). Processing of the structure was done with Jmol application, an open-source Java viewer for chemical structures in 3D. **(a)** CR4CR5 aptamer (CR4/CR5 domain). **(b)** CR7 aptamer (CR7 fork of ScaRNA domain). **(c)** AA aptamer (P6.1 fork). **(d)** AAmut aptamer (P6.1 fork with G194A mutation). **(e)** MDS aptamer (P5 and P4.2 fork). **(f)** MDSmut aptamer (P5 and P4.2 fork with G206U and A208U mutations).

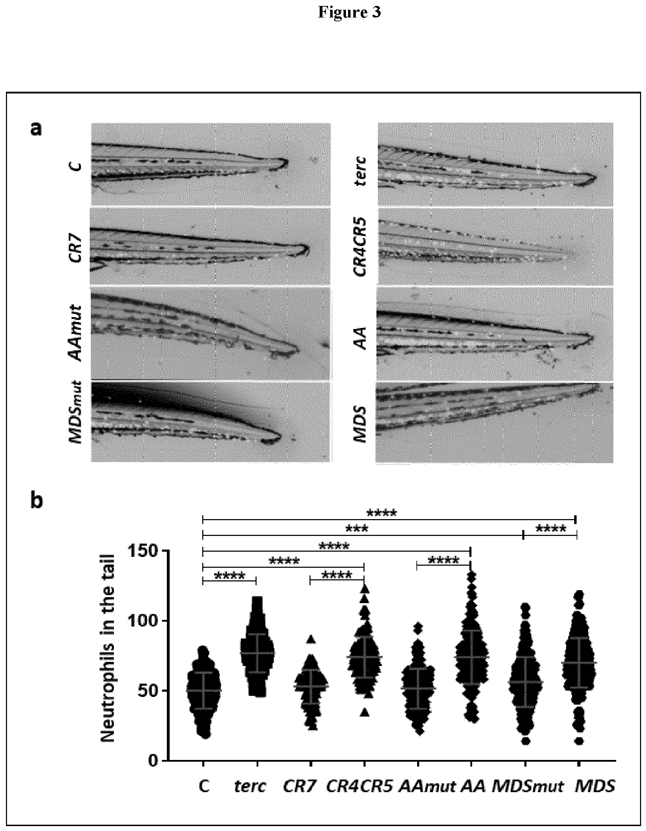

**Figure 3****. Aptamers increase neutrophil number in zebrafish.** Zebrafish embryos were microinjected with terc or CR7, CR4CR5, AA, AAmut MDS and MDSmut aptamers. **(a)** Representative pictures of neutrophils in the tail of 48 hours post-fertilization (hpf) larvae. **(b)** Neutrophil number quantification in the tail of 48 hpi larvae. Data are average of 12 independent experiments. Each dot represents the number of neutrophils from a single larva, while mean ± SEM for each group is also shown. ***p < 0.001 and ****p < 0.0001 according to ANOVA followed by Dunnett multiple range test.

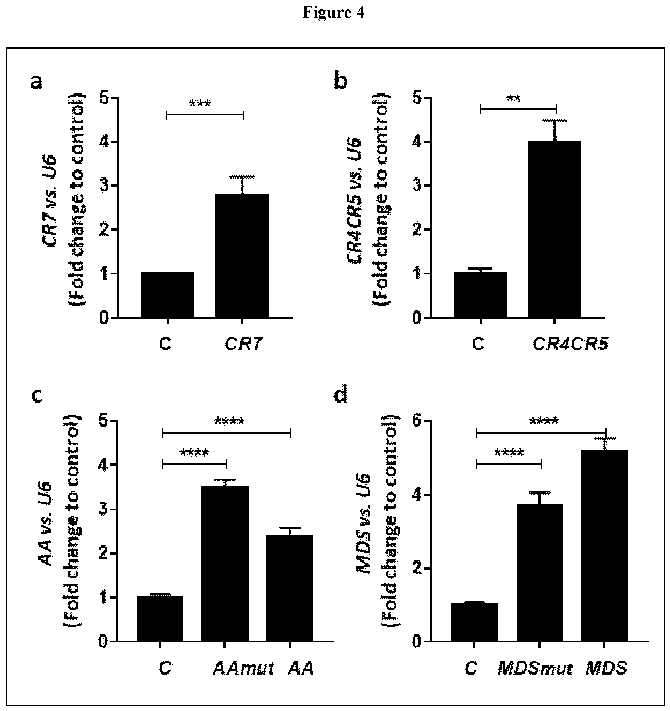

**Figure 4****. Aptamers quantification.** Levels of CR7 **(a),** CR4CR5 **(b),** AA and AAmut **(c)** and MDS and MDSmut **(d)** aptamers were measured by RT-qPCR. The levels were normalized to U6 gene and relative to control. Data are representative results of at least 3 independent experiments; n > 20 larvae per group. **p < 0.01, ***p < 0.001 and ****p < 0.0001 according to Student's t test for two samples (a, b) and ANOVA followed by Dunnett multiple range test for three samples **(c, d).**

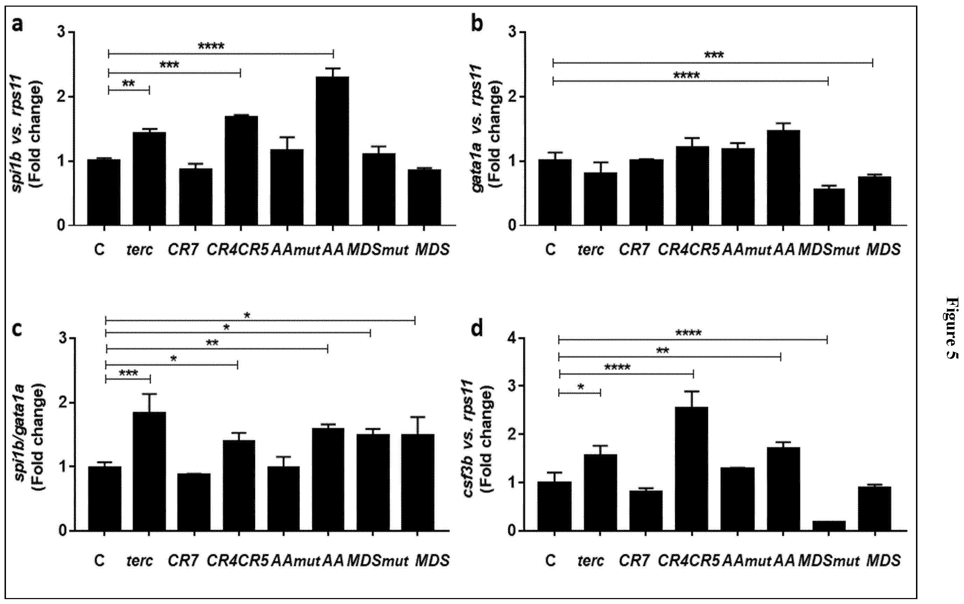

**Figure 5****. Aptamers increased csf3b and spilb transcript levels.** mRNA levels of the myeloid genes spilb **(a),** gatala **(b)** and csf3b **(d),** and spi1b/gata1a ratio **(c)** of 48 hpf larvae microinjected with indicated aptamers analysed by RT-qPCR. The expression was normalized to rps11 and relative to control sample. Data are representative of at least 3 independent experiments; n > 20 larvae per group. *p < 0.05, **p < 0.01, ***p < 0.001 and ****p < 0.0001 according to ANOVA followed by Dunnett multiple range test.

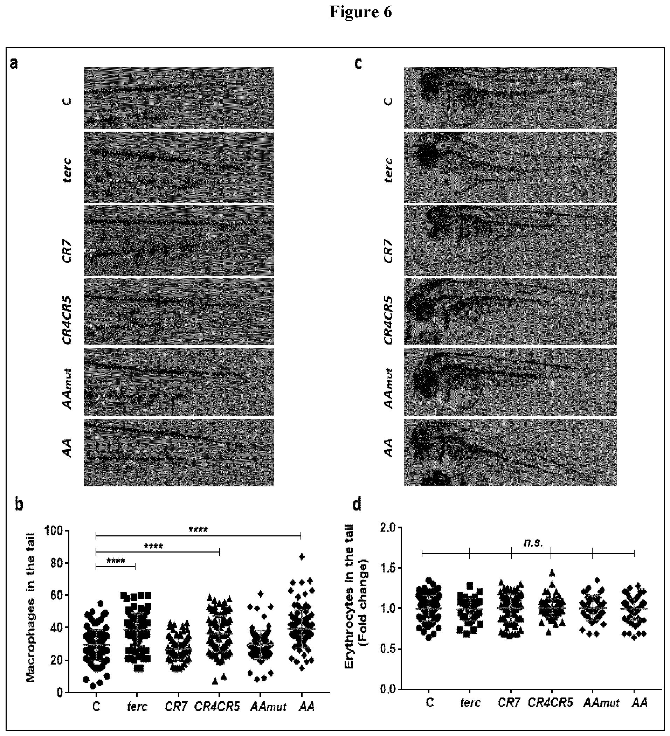

**Figure 6****. Aptamers affect monocyte, but not erythrocyte lineages in zebrafish.** mpeg1:GFP and lcr:GFP transgenic zebrafish embryos were microinjected with full terc and indicated aptamers. **(a)** Representative images of 48 hpf mpeg1:GFP larvae. **(b)** Quantification of the macrophages number in zebrafish tail. **(c)** Representative images of 48 hpf lcr:GFP larvae. **(d)** Quantification of erythrocytes number in zebrafish tail by total fluorescence intensity measurement after normalization with the control. Data are average of 3 independent experiments. Each dot represents the number of macrophages or erythrocytes from a single larva, while mean ± SEM for each group is also shown. n.s., not significant; ****p < 0.0001 according to ANOVA followed by Dunnett multiple range test.

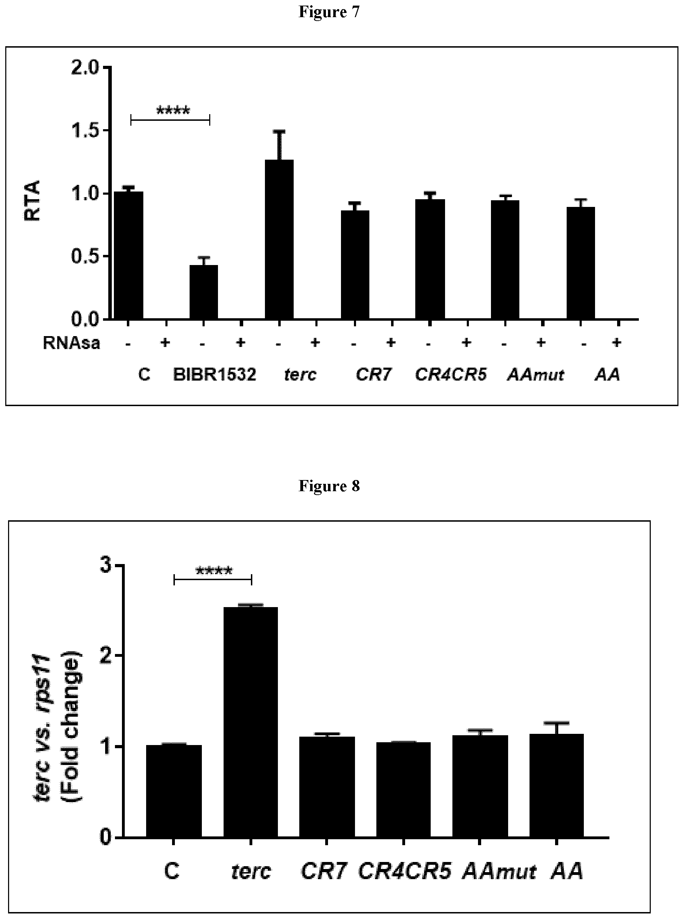

**Figure 7****. Aptamers do not affect telomerase activity.** Zebrafish embryos were microinjected with BIBR1532 (telomerase inhibitor), full terc and the indicated aptamers. Larvae were collected at 48 hpf and the relative telomerase activity (RTA) was measured by qPCR in protein extracts. Data are shown as mean + s.e.m. of 2 independent experiments. ****p < 0.0001 according to ANOVA followed by Dunnett multiple range test.
**Figure 8****. Aptamers did not affect terc transcript levels.** terc RNA levels from 48hpf larvae microinjected with full terc or the indicated aptamers were measured by qPCR. Gene expression was normalized to rps11 and then to the control sample. Data are representative results of 3 independent experiments; n > 20 larvae per group. ****p < 0.0001 according to ANOVA followed by Dunnett multiple range test.

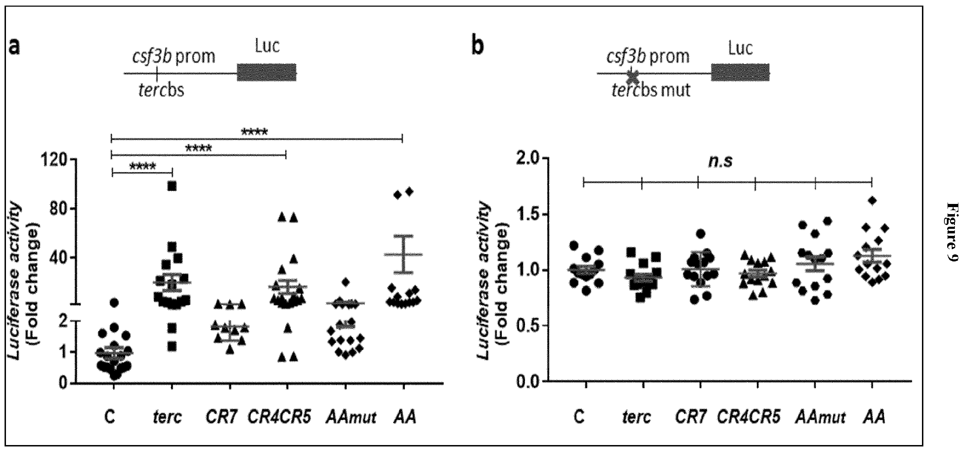

**Figure 9****. Aptamers control the activity of myeloid gene promoters in a terc binding site-dependent manner.** Zebrafish embryos were microinjected with a pCMVRenilla plasmid, a firefly luciferase reporter containing the wild type **(a)** or the mutant tercbs **(b)** of csf3b promoter and the indicated aptamers. At 48 hours, luciferase activity was measured in larval protein extracts. Luciferase activity was normalized to Renilla activity and the results shown as fold change relative to control. Data are representative of three independent experiments. Each dot represents the luciferase activity of a pool of 10 larvae, while mean ± SEM for each group is also shown. n.s., not significant; ****p < 0.0001 according to ANOVA followed by Dunnett multiple range test.

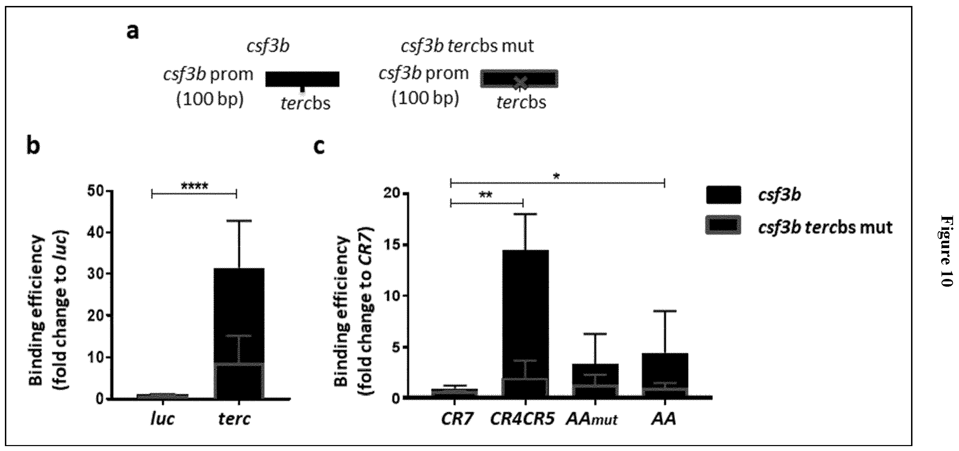

**Figure 10****. *In vitro* aptamer-DNA binding capacity. (a)** Schematic drawing of dsDNA probes of csf3b upstream regulatory sequences (100 bp) containing wild type or mutant tercbs used in the in vitro DNA binding assays. **(b, c)** RT-qPCR of in vitro DNA-binding assay eluates. For each probe, data are the average of 2 independent experiments. luc, luciferase RNA. All data are shown as mean + s.e.m. *P< 0.05, ** P< 0.01 and **** P< 0.0001 according to Student's t test for two samples **(b)** and ANOVA followed by Bonferroni multiple range test for three samples **(c).**

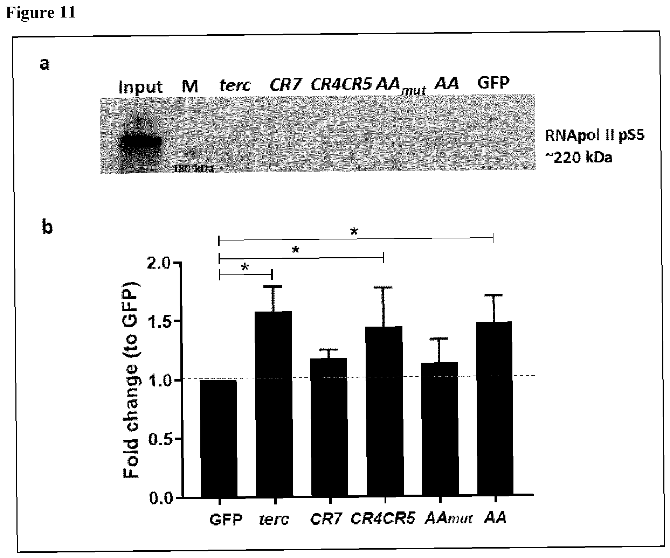

**Figure 11****. Aptamer RNA pulldown experiment. (a)** Western blot of aptamer RNA pulldown eluates using anti-phospho-serine 5 RNA Pol II antibody (220 kDa). Marker (M) is show in lane 2. **(b)** Quantification of aptamers interaction with RNA Pol II using Quantity One software; n = 3. All data are mean + s.e.m. *P< 0.05 for one-way analysis of variance (ANOVA) plus Bonferroni post-test.

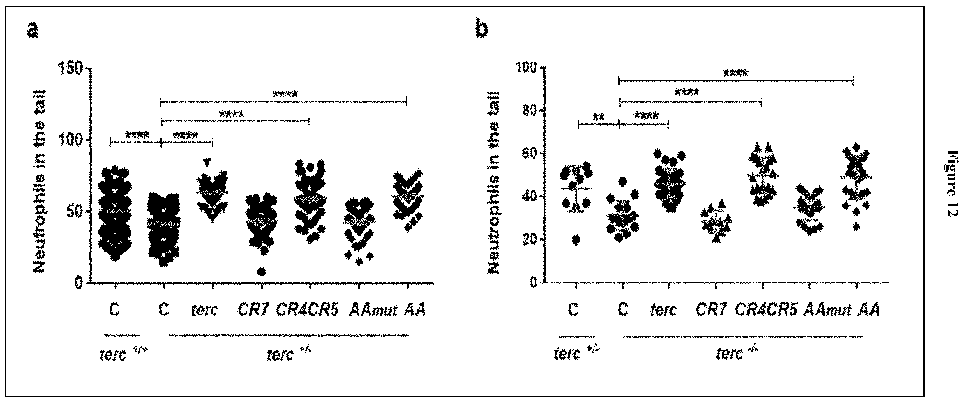

**Figure 12****. Aptamers increase neutrophil number in terc-deficient model. (a)** terc +/embryos (from mpx:GFP crossed with terc knockout (terc -/-)) were microinjected with aptamers. At 48 hpf, neutrophil number (GFP positive cells) in the tail were quantified by fluorescence. **(b)** terc -/- embryos (from two terc -/- parental crossed each other) were microinjected with aptamers. At 48 hpf, larvae were stained with TSA reagent, which specifically marks neutrophils with green fluorescence, and then quantified. Data are average of 4 independent experiments. Each dot represents the number of neutrophils from a single larva, while mean ± SEM for each group is also shown. **p < 0.01 and ****p < 0.0001 according to ANOVA followed by Tukey multiple range test.

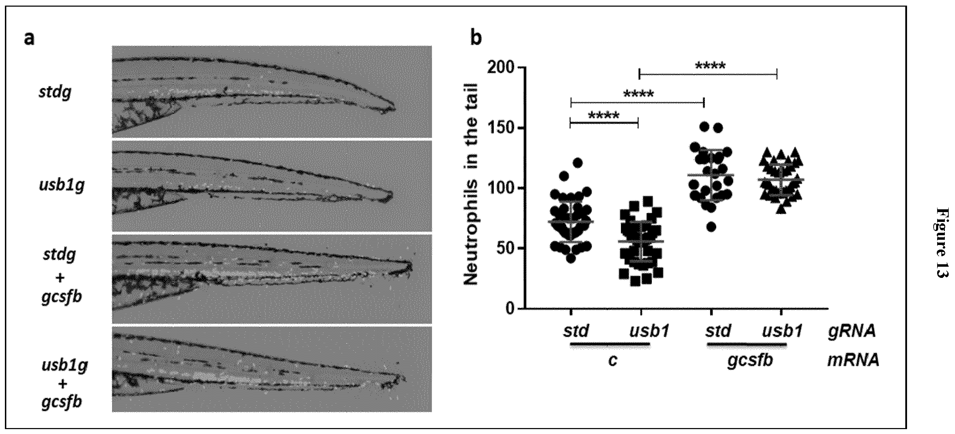

**Figure 13****. usb1 zebrafish model. (a)** Representative pictures of 3 dpflyz:dsRED zebrafish larvae microinjected with usb1 or std gRNA plus recombinant Cas9 (as a control) in presence or absence of gcsfb mRNA. **(b)** Neutrophils (dsRED positive cells) quantification in the tail at 72 hpf in the different conditions. Data are average of 2 independent experiments. Each dot represents the number of neutrophils from a single larva, while mean ± SEM for each group is also shown. ****p < 0.0001 according to ANOVA followed by Tukey multiple range test.

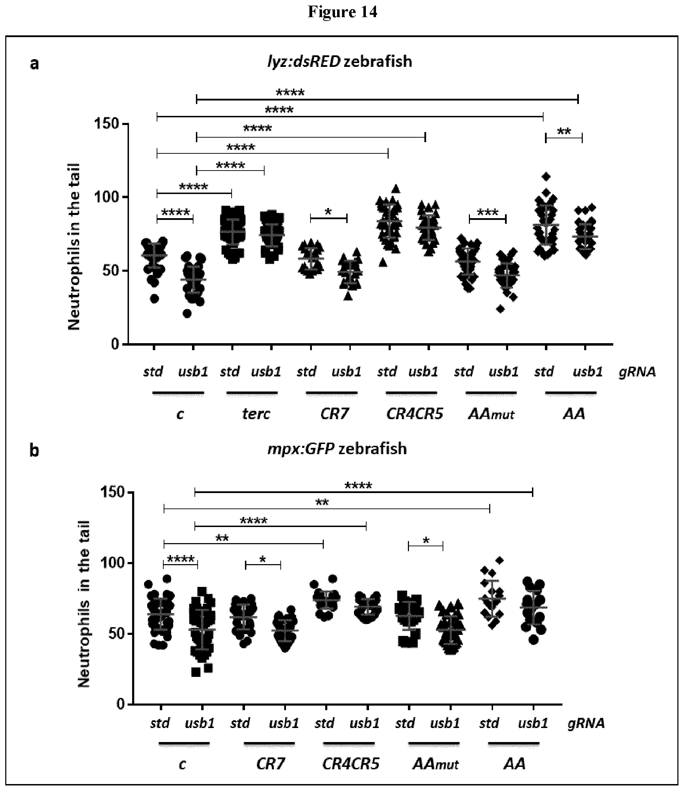

**Figure 14****. Aptamers in usb1 zebrafish model.** Tg(lyz:dsRED) (a) or Tg(mpx:eGFP) **(b)** zebrafish embryos were microinjected with **usb1** or std gRNAs plus recombinant Cas9 in the presence or absence of terc or the indicated aptamers. At 72 hpf, neutrophil number in the tail were quantified. Data are average of 2 independent experiments. Each dot represents the number of neutrophils from a single larva, while mean ± SEM for each group is also shown. *p < 0.05, **p < 0.01 and ****p < 0.0001 according to ANOVA followed by Tukey multiple range test.

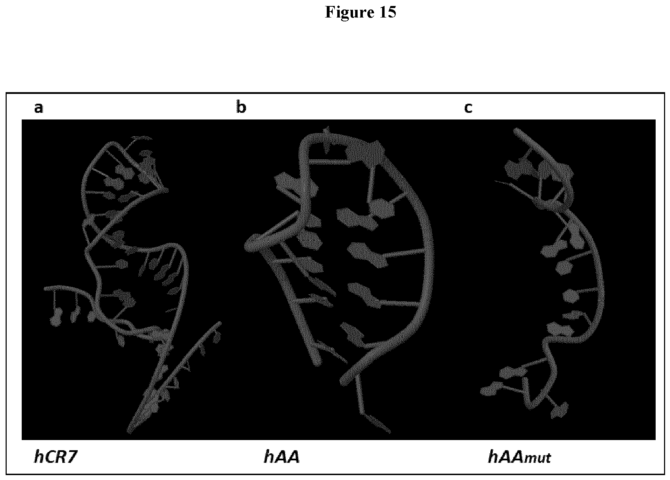

**Figure 15****. 3D human aptamer structure.** Prediction and modelling of tertiary RNA structure using the RNAComposer system (http://rnacomposer.cs.put.poznan.pl/). The processing of the structure was done with Jmol application, an open-source Java viewer for chemical structures in 3D. **(a)** hCR7 aptamer (P8a plus P8b domains). **(b)** hAA aptamer (P6.1 fork). **(c)** hAAmut aptamer (P6.1 fork with G319A mutation).

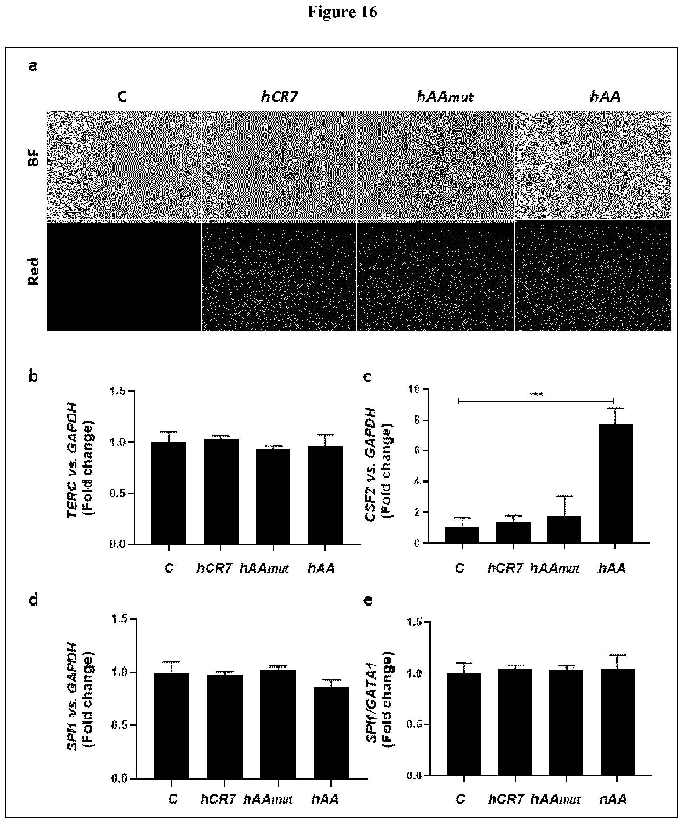

**Figure 16****. Effects of human aptamers in HL60 cell line.** HL60 cells were treated with aptamers for 48 hours. **(a)** Representative phase contrast and red fluorescence images of HL60 cells after 24h of culture. Note that cells treated with hAA and hAAmut aptamers showed red fluorescence. **(b,c)** Transcript levels of CSF2 **(c)** and SPI1 **(d)** in control and aptamer-treated cells. The gene expression was normalized to GAPDH and then to control cells. Data are representative of 2 independent experiments. n.s., not significant; ****p < 0.0001 according to ANOVA followed by Dunnett multiple range test.

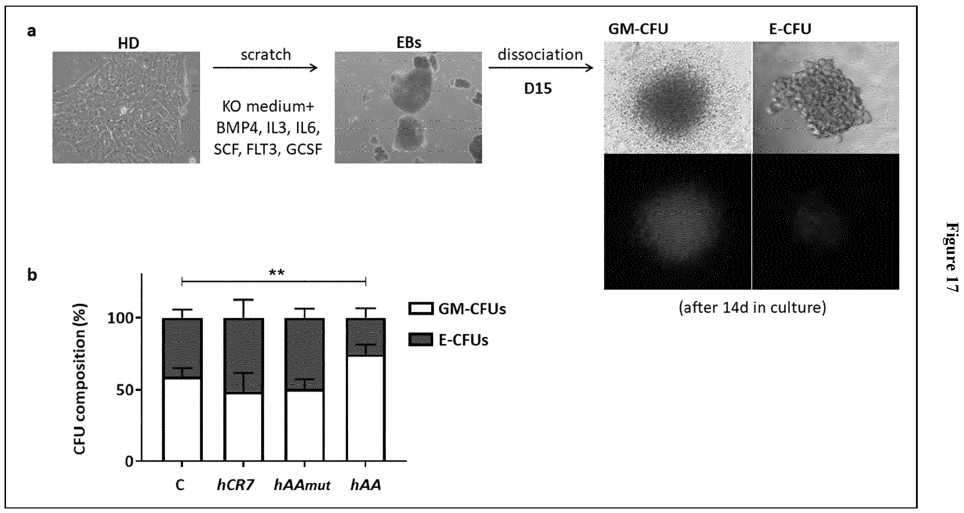

**Figure 17****. CFU assay from healthy donor (HD) iPS cell line treated with aptamers. (a)** Workflow for differentiation of iPS cells. **(b)** Composition of granulocyte/monocyte (GM-CFUs) and erythroid (E-CFUs) colonies in CFU assays, using EBs derived from the HD iPS cell line. Data are shown as mean + s.e.m. (n = 6 independent EB formation experiments). **p < 0.01 according to Fisher's Exact Test.

### Detailed description of the invention

The present invention is illustrated by means of the Examples set below without the intention of limiting its scope of protection.

### Example 1. Material and methods

### Example 1.1. Animals.

Zebrafish (*Danio rerio* H.) specimens were obtained from the Zebrafish International Resource Centre and mated, staged, raised and processed using standard procedures (Widrick et al., 2018). The transgenic lines *roy*^{*a9*/}*^{a9}; nacre*^{*w2*/*w2*} (Casper for simplicity) (White et al., 2008), *Tg(mpx:::eGFP)*^{il13} (*mpx:GFP* for simplicity) (Renshaw et al., 2006), *Tg(mpeg1:eGFP)^{gl22} (mpeg1:GFP* for simplicity) (Ellett et al., 2011), *Tg(lcr:eGFP)^{cz3325} (lcr:GFP* for simplicity) (Ganis et al., 2012) and *Tg(lyz:dsRED)^{nz50} (lyz:dsRED* for simplicity) (Hall et al., 2007), were previously characterized.

### Example 1.2. Aptamers and RNA injection.

Aptamers were synthesized as a single-stranded RNA oligos unmodified or with modifications of Cy3 or biotin at the 3'end by Sigma-Aldrich, or by Horizon Discovery in the case of the *CR4CR5* aptamer, with HPLC purification. *terc* and *csf3b* RNAs were *in vitro* transcribed with the mMESSAGE mMACHINE kit (Ambion) and purified with phenol chloroform. Both mRNA and aptamers were mixed in a microinjection buffer (0.5 × Tango buffer and 0.05% phenol red solution) and microinjected into the yolk sac of one cell stage embryos using a Narishige IM300 microinjector (0.5-1 nl per embryo). The amount of *terc* and *csf3b* injected was 200 and 100 pg/egg, respectively. The amount of aptamers injected was 605 pg/egg for *CR4CR5,* 337 pg/egg for CR7, 337 pg/egg for *MDS* and *MDSₘᵤₜ,* and 138 pg/egg for *AA* and *AAₘᵤₜ,* in order to achieve the same number of molecules per egg in all the aptamers used.

### Example 1.3. Zebrafish blood cell line count.

Eggs of 1-8 cell stage from the transgenic lines *mpx:GFP, mpeg1:GFP, lcr:GFP* and *lyz:dsRED* were microinjected with the different aptamers or *terc.* At 48 or 72 (in *usb1-*CRISPR experiments) hours post-injection, the neutrophils (*mpx:GFP* or *lyz:dsRed* positive cells) and the macrophages (*mpeg1:eGFP* positive cells) of the tail region were counted under a Leica M205 FA fluorescence stereo microscope. To quantify the erythrocytes (*lcr:GFP* positive cells), the larvae were photographed with Leica M205 FA fluorescence microscope equipped with a DFC365FX camera (Leica) and the total fluorescence intensity of the tail region was quantified with Leica Application Suite X (LAS X) software.

### Example 1.4. Neutrophil staining.

Zebrafish embryos were fixed 2 hours at room temperature (RT) in 4% paraformaldehyde (PFA). Fixed larvae were briefly washed twice in PBS and incubated in 1:50 TSA Cyanine5 (TSA TM-Plus Fluorescein kit, Perkin Elmer, UK) without light for 10 minutes at 28°C. Neutrophils were specifically fluorescein stained and then counted under a Leica M205 FA fluorescence stereo microscope equipped with a DFC365FX camera (Leica).

### Example 1.5. Aptamer quantification.

Total RNA was extracted from larvae tails with TRIzol reagent (Thermo Fisher Scientific) using Direct-zol RNA Miniprep kit (Zymo Research) and treated with DNAsa following the manufacturer's instructions. cDNA was generated with the miScript II RT kit (Qiagen), following the manufacturer's instructions. Real-time qPCR was performed with a StepOnePlus instrument (Applied Biosystems), using miScriptSYBR Green PCR kit (Perfect Real Time) (Qiagen), containing a reverse common primer. Reaction mixtures were incubated for 10 min at 95 °C, followed by 40 cycles of 15 s at 95 °C, 1 min at 60 °C, and finally 15 s at 95 °C, 1 min at 60 °C and 15 s at 95 °C. For each sample, gene expression was normalized to U6 snRNA (the most commonly housekeeping gene used as reference in miRNA quantification (Peltier and Latham, 2008)), using the comparative Ct method (2-ΔCt). The forward primers used are shown in **Table 1.** In all cases, each PCR was performed with triplicate samples and repeated in every single aptamer microinjection experiment.

**Table 1**

| **Name** | **Species** | **Sequence (5'-3')** |
|---|---|---|
| ***CR4CR5*** | zebrafish | SEQ ID NO: 4 |
| *CR7* | zebrafish | SEQ ID NO: 5 |
| ***AA*/*AAₘᵤₜ*** | zebrafish | SEQ ID NO: 6 |
| ***MDS*/*MDSₘᵤₜ*** | zebrafish | SEQ ID NO: 7 |
| ***U6*** | zebrafish | "Qiagen property" |

| | | |
|---|---|---|
| *Primers used for aptamer qPCR quantification.* | | |

### Example 1.6. Gene expression analysis.

Total RNA was extracted from larvae tails with TRIzol reagent (Thermo Fisher Scientific) using Direct-zol RNA Miniprep kit (Zymo Research) following the manufacturer's instructions. RNA was treated with DNase I, RNAsa free (Qiagen) on the column. SuperScript^{™} VILO^{™} cDNA Synthesis Kit (Invitrogen) was used to synthesize first-strand cDNA following the manufacturer's instructions. Real-time PCR was performed with a StepOnePlus instrument (Applied Biosystems) using SYBR^{®} Premix Ex Taq^{™} (Perfect Real Time) (Takara). Reaction mixtures were incubated for 30 seconds (sec) at 95°C, followed by 40 cycles of 5 sec at 95°C, 20 sec at 60°C, and finally a melting curve protocol. Ribosomal protein S11 (*rps11*) or glyceraldehyde 3-phosphate dehydrogenase (*GAPDH*) content in each sample was used for normalization of zebrafish or human mRNA expression, respectively, using the comparative Ct method (2-ΔCt). The primers used are shown in **Table 2.** In all cases, each PCR was performed with triplicate samples and repeated, at least, with two independent samples.

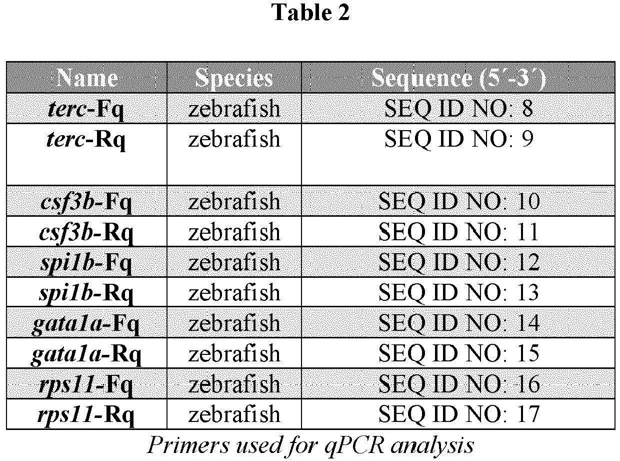

### Example 1.7. Telomerase activity assay

A real-time quantitative telomeric repeat amplification protocol (Q-TRAP) analysis was performed to measure telomerase activity (Herbert et al., 2006). Briefly, proteins were extracted from whole 2 days post-fertilization (dpf) larvae, previously microinjected with RNA, aptamers or 20 µM BIBR1532 (telomerase inhibitor, Santa Cruz Biotechnology, #203843) in each case, using ice-cold TRAPeze IX CHAPS Lysis Buffer (Sigma Aldrich). A real-time Q-TRAP was performed with 0.1 µg protein extracts. To make the standard curve, a 1:10 dilution series of positive telomerase sample (HeLa cells) was used. Control samples were obtained by treating the cell extracts with 1 µg RNase at 37°C for 20 min. Data was collected and converted into Relative Telomerase Activity (RTA) units by calculating: RTA of sample=10^{(Ctsample-γint)/slope}. The standard curve obtained was y=-3.2295x+23.802.

### Example 1.8. In vivo luciferase assay.

pCMV-Renilla plasmid, containing Renilla luciferase (Promega) and the pGL3basic vector constructs containing the *csf3b* promoter region of zebrafish followed by the firefly luciferase, were microinjected together with the indicated aptamers or *terc* into the yolk sac of the single-cell stage embryos. After 48 hours, tail sections were obtained, pooled (10 tails per pool), homogenized with a pellet pestle and centrifuged for 3 min at 13,000 rpm to remove cellular debris. Then, the extracts were assayed for firefly and Renilla luciferase activity using the Dual Luciferase Reporter Assay System (Promega) on a Luminometer Optocomp I (MGM Instruments). The results were normalized with the Renilla activity (Alcaraz-Perez et al., 2008).

### Example 1.9. In vitro DNA binding assay.

One hundred pb sense and antisense 3' biotin-tagged-DNA probes of the zebrafish *csf3b* promoter region encompassing the *terc*-binding sites (SIGMA-Aldrich) were designed to measure the binding capacity of *terc* or aptamers to these sequences. To perform the annealing, 25 µM of each probe were incubated in annealing buffer (10 mM Tris-HCl pH 7.5, 200 mM NaCl, 0.2 mM EDTA) at 95°C for 4 min, 10 min at 70°C and they were slowly cooled down to RT for 20 min to allow annealing. Then, the dsDNA probes were bound to 10 µl of Dynabeads MyOne Streptavidin C1 magnetic beads (Invitrogen) for 15 min at RT. Probe excess was removed by washing beads 2 times with annealing buffer for 5 min. After that, 50 ng of luciferase (Promega), *terc* and aptamers were added and incubated at RT for 30 min in rotation. The beads-dsDNA-RNA complexes were washed 3 times at RT for 10 min and RNA was eluted by incubation in water at 95°C for 5 min. The eluted RNAs were reverse transcribed with SuperScript IV VILO Master Mix (Invitrogen) or mi Script II RT kit (Qiagen) following the manufacturer's instructions, for *terc* and luciferase or for aptamers, respectively. The samples were subjected to quantitative-PCR (qPCR) for luciferase, *terc* and aptamers detection, under the same conditions. To be able to compare the expression cycles of *terc,* aptamers and luciferase, a previous normalization was made. Normalization consisted of a primers efficiency calibration curve starting from 1 ng/µl of RNA, and serial dilutions of 0.1 ng/µl, 0.01 ng/µl and 0.001 ng/µl of every RNA. Luciferase primers are more efficient than *terc* primers in 1.3 cycles, CR7 aptamer in 3.8 cycles, *CR4CR5* aptamer in 19.2 cycles and *AA* and *AAₘᵤₜ* aptamers in 18.7 cycles.

### Example 1.10. RNA pulldown.

RNA pulldown experiments were performed as described (Tsai et al., 2010), with some modifications. Biotin-labelled RNAs were *in vitro* transcribed using the Biotin 3' End DNA Labeling Kit (Thermo Scientific), to incorporate 1-3 biotinylated ribonucleotides onto the 3' end of DNA/RNA strands, following manufacturer's instructions. Three micrograms of biotinylated RNA were heated to 70°C for 5 min and put on ice for 2 min. An equal volume of 2x RNA structure buffer (20 mM Tris pH 7, 0.2 M KCl, 20 mM MgCl₂) was added and then shifted to RT to allow RNA secondary structure formation. The folded RNAs were incubated for 1 hour at 4 °C with rotation with 60 µl of washed Dynabeads MyOne Streptavidin C1 magnetic beads (Invitrogen). Larvae of 5 dpf were anesthetized and the protein extract was obtained by homogenization in RIP buffer (25 mM Tris pH 7.4, 150 mM KCl, 0.5 mM DTT, 0.5% NP-40). The protein extract was centrifuged for 20 min at 4°C and the supernatant was pre-cleared 1h with 30 µl of beads at 4°C in rotation. Then 3 miligrams of pre-cleared protein were incubated with the biotinylated RNAs-beads complexes for 4h at 4°C with rotation. The complexes were magnet-captured, washed with RIP buffer five times at 4°C for 5 min and boiled in 2x Laemmli buffer (Sigma) at 90°C for 10 min for protein elution. The eluted proteins were subjected to polyacrylamide gel electrophoresis, wet transferred to a nylon membrane (GE Healthcare) and analysed by Western blot. The membranes were incubated for 1 h with TTBS (Tween-Tris-buffered saline) containing 5% (w/v) skimmed dried milk powder and immunoblotted using anti-phospho-Serine 5 RNA polymerase II CTD repeat YSPTSPS mouse antibodies (dilution 1:1000) 16 h at 4°C (pS5 RNA pol II, ab5408, Abcam). The blots were then washed with TTBS and incubated for 1 h at room temperature with the secondary HRP-conjugated antibody (dilution 1:1000) in 5% (w/v) skimmed milk in TTBS. After repeated washes, the signal was detected with the enhanced chemiluminescence reagent and ChemiDoc XRS (Biorad).

### Example 1.11. Zebrafish poikiloderma with neutropenia model.

The usefulness of the aptamers to reverse neutropenia was evaluated in a zebrafish model of poikiloderma with neutropenia (Patil et al., 2015, Colombo et al., 2015), an inherited disorder caused by loss-of-function mutations in *USB1* (U6 SnRNA Biogenesis Phosphodiesterase 1) gene, which encodes a conserved phosphodiesterase that regulates the stability of spliceosomal U6-RNA. The CRISPR RNA (crRNA) obtained from IDT with the following target sequence was used: *usbl* 5'-SEQ ID NO: 18-3'. It was resuspended in duplex buffer at 100 µM and 1 µl was incubated with 1 µl of trans-activating CRISPR RNA (tracrRNA, 100 µM) at 95°C for 5 min and 5 min at room temperature to form the complex. One µl of this complex was mixed with 0.25 µl of recombinant Cas9 (10 mg/ml) and 3.75 µl of duplex buffer. The crRNA mix was then microinjected in the yolk sac of zebrafish one-cell stage embryos using a microinjector (Narishige) (1 nl per embryo). Neutrophils were counted at 3 dpf as indicated above.

### Example 1.12. HL60 cell culture.

The human promyelocytic HL60 cell line was cultured in RPMI-1640 medium (Lonza) supplemented with 10% FCS, 2 mM glutamine and 1% penicillin-streptomycin. Cells were maintained at 37°C with 5% CO₂ and split before confluence every 72h.

### Example 1.13. iPS cell culture, differentiation towards hematopoietic lineage and colony-forming unit (CFU) assay.

iPS cell lines were maintained undifferentiated in a 6mm plates treated with Matrigel (Corning) and mTeSR^{™} Plus medium (Stem Cell Technologies). Media was changed daily or every two days, and the cells were split weekly by dissociation with 200 U/ml of collagenase IV (Invitrogen). iPS cell culture were visualized daily by phase-contrast microscopy.

For hematopoietic differentiation, undifferentiated iPS cells at 70-80% confluence were treated with Matrigel (Corning) 24 hours before starting the differentiation. To generate the Embrionic Bodies (EBs), the iPS cells were treated with collagenase IV and scraped of the attachments. They were then transferred to 6 wells low-attachment plates (Corning) to allow EBs formation by incubation in differentiation medium consisting of KnockOut^{™} Dulbecco's modified Eagle's medium (ThermoFisher) supplemented with 20% non-heat-inactivated fetal bovine serum, 1% nonessential amino acids, 1 mM glutamine and 0.1 mM β-mercaptoethanol. The medium was changed the next day (day 1) with the same differentiation medium supplemented with hematopoietic cytokines: 300 ng/ml stem cell factor (R&D), 300 ng/ml FMS-like tyrosine kinase-3 ligand (R&D), 10 ng/ml IL-3 (R&D), 10 ng/ml IL-6 (R&D), 50 ng/ml granulocyte colony-stimulating factor (R&D) and 25 ng/ml bone morphogenetic protein-4 (Miltenyi) (Wang et al., 2004, Menendez et al., 2004, Wang et al., 2005). EBs were dissociated using collagenase B (Roche Diagnostic) for 2 hours at 37°C followed by 10 minutes incubation at 37°C with enzyme-free Cell Dissociation Buffer (Invitrogen) at day 15 of development. Single-cell suspension was obtained by gentle pipetting and passage through a 70-µm cell strainer (Becton Dickinson).

CFU assays were performed by plating 100,000 cells from EBs at day 15 into methylcellulose H4434 culture medium (Stem Cell Technologies). Cells were incubated at 37°C in a 5% CO₂-humidified atmosphere and colonies counted at day 14 of the CFU assay using standard morphological criteria (Bueno et al., 2009, Menendez et al., 2001, Catalina et al., 2009). The iPS cell workflow is depicted in **Figure 17****.**

### Example 2. Results.

### Example 2.1. Aptamers design.

Telomerase RNA plays an essential role in myelopoiesis by increasing the expression of myeloid genes. CR4/CR5 domain of *TERC* is essential in this non-canonical function, since mutations of DC patients affecting this domain prevent activation of myelopoiesis, while mutations in the template or ScaRNA domain did not affect myelopoiesis (Garcia-Castillo et al., unpublished). According to this knowledge, several aptamers based on the CR4/CR5 *terc* domain of zebrafish were designed to test its potential clinical utilities in blood disorders. Aptamers are single-chain oligonucleotides, capable of recognizing specifically and with high affinity target molecules through a three-fold folding of their chain. They are small, cheap, easy to synthesize and have no immunogenicity *in vivo.* This makes them ideal candidates for therapeutic applications (Zhu and Chen, 2018).

Since many *TERC* mutations have been mapped and its clinical manifestation reported (reviewed by (Carroll and Ly, 2009)), we focus on those mutations affecting the CR4/CR5 domain that cause blood diseases. Thus, DC patients with mutations in position G305A have aplastic anaemia (AA), while those with mutations in G322A and G323T show myelodysplastic syndrome (MDS) **(****Figure 1****).**

The following aptamers to be preclinically tested in zebrafish model (Martin et al., 2011, Gore et al., 2018) were designed:
i) *CR4CR5:* Aptamer based on CR4/CR5 *terc* domain with 79 nucleotides (SEQ ID NO: 19). Its predicted 3D structure is shown in **Figure 2a****.**
ii) *AA:* Aptamer consisting of only of P6.1 fork of CR4/CR5 domain with 17 nucleotides (SEQ ID NO: 20) **(****Figure 2c****).** This fork has been selected, as has been mentioned before, because the mutation G305A in humans (G194A in zebrafish) has been shown to produce AA (Carroll and Ly, 2009) **(****Figure 1****).** As a control, another aptamer was designed from the same fork sequence but containing the mutation found in DC patients (SEQ ID NO: 21), so it was called as *AAₘᵤₜ.* Notably, this mutation drastically altered its structure **(****Figures 2c** and **2d****),** suggesting the importance of the tertiary structure of *terc* to perform its non-canonical function in myelopoiesis and anticipating its relevance in DC.
iii) *MDS:* This aptamer consists of part of P4.2 and P5 subdomains of CR4/CR5 domain and has 44 nucleotides (SEQ ID NO: 22). In P5 subdomain there are two mutations (G322A and G323T in humans and G206U and A208U in zebrafish) found in DC patients that develop MDS (Carroll and Ly, 2009) **(****Figure 1****).** As a control aptamer, the *MDSₘᵤₜ* was designed with the patient mutations (SEQ ID NO: 23). Surprisingly, these mutations did not strongly alter the predicted structure of the aptamer **(****Figure 2f****)**.
iv) CR7: This is an additional control aptamer consisting in the CR7 fork, containing the P8 fork and is 46 nucleotides long (SEQ ID NO: 24) **(****Figure 2b****).** It was designed based in a different domain of *terc,* ScaRNA domain, which is not involved in myelopoiesis since the mutation of this domain found in DC patient was able to increase the number of neutrophils as wild type *terc* did.

### Example 2.2. Aptamers were able to increase neutrophil number in zebrafish.

The transgenic zebrafish line *mpx:GFP,* whose neutrophils have a GFP expression driven by the *mpx* promoter, was used to evaluate the effect of aptamers in myelopoiesis. Aptamers were microinjected into zebrafish embryos at one-cell stage and 48 hours later the neutrophils (GFP positive cells) were quantified **(****Figure 3****).** Surprisingly, all aptamers based in the wild type *terc* sequence, i.e. *CR4CR5, MDS* and *AA,* were able to increase neutrophil number at the level of full *terc.* This result support the idea that small domains of *terc* can perform its function in myelopoiesis as the full molecule. However, CR7 aptamer failed to increase neutrophil number, confirming the specificity of effects observed with *CR4CR5, MDS* and *AA* aptamers. More importantly, *AAₘᵤₜ* aptamer did not affect the number of neutrophils in zebrafish, demonstrating that this point mutation had a drastic effect in AA function, suggesting the importance of a correct tertiary structure for its function in regulation of myelopoiesis and confirming the relevance of this mutation in DC pathology. Surprisingly, *MDSₘᵤₜ* slightly, but significantly, increased neutrophil number, perhaps because of the weak effect of this mutation in tertiary structure of the aptamer **(****Figure 2f****).**

The stability and amount of aptamer and *terc* in zebrafish were quantified by RT-qPCR. Similar levels were achieved of all aptamers **(****Figure 4****).** In all successive experiments, levels of aptamers in the larvae were always measured and if they were not expressed of reached different levels (more than 2-fold change), the experiment was discarded.

### Example 2.3. Aptamers regulate myeloid gene expression in zebrafish.

As *terc* regulates neutrophil number in zebrafish larvae through fine-tuning *spi1b* and *csf3b* mRNA expression (Alcaraz-Perez et al., 2014), the expression of these genes was analysed in larvae treated with the different aptamers. The results showed that although *CR4CR5* and *AA* aptamers were able to increase *spi1b* and *csf3b* transcript levels as full *terc,* CR7 and *AAₘᵤₜ* negative controls failed to do so **(****Figure 5****),** confirming the results obtained with the number of neutrophils **(****Figure 3****).** However, in the case *of MDS* and its control (*MDSₘᵤₜ*), *spi1b*/*gata1a* balance increased but, curiously, because both decreased *gata1a* mRNA levels. In addition, *MDS* and *MDSₘᵤₜ* did not increase *csf3b* mRNA levels **(****Figure 5d****).**

We decided to exclude from the study both *MDS* and *MDSₘᵤₜ* aptamers and focus in *CR4CR5* and *AA* aptamers, since functional and molecular characterization of each aptamer was time consuming and expensive.

### Example 2.4. Aptamers affect monocyte, but not erythrocyte lineage in zebrafish.

The above results demonstrated that aptamers were able to increase the number of neutrophils in zebrafish model, but we wanted to know if other bloodlines were also affected. In order to study if the addition of aptamers affected either monocyte or erythrocyte lineages, *mpeg1:GFP* and *lcr:GFP* zebrafish transgenic lines were used. On one hand, similar increased numbers of macrophages were observed in larvae treated with *CR4CR5* and *AA* aptamers and full *terc* **(****Figures 6a, 6b****).** As expected from previous experiments, CR7 and *AAₘᵤₜ* control aptamers were unable to increase the number of macrophages **(****Figures 6a, 6b****).** On the other hand, there were no differences in erythrocyte number among larvae microinjected with different aptamers, including control aptamers. **(****Figure 6c, 6d****).** *terc* did not change either the number of erythrocytes, confirming previous results (Alcaraz-Perez et al., 2014). These results indicate that aptamers affect myelopoiesis but not erythropoiesis. This is a crucial point for their possible therapeutic uses.

### Example 2.5. Aptamers do not affect telomerase activity in zebrafish.

In order to study aptamers mechanism of action, it is interesting to know if they affect telomerase. As a control, same amount of a telomerase activity inhibitor (BIBR 1532), which should reduce the telomerase activity, was microinjected. The results showed that while neither aptamers nor full *terc* affect telomerase activity, telomerase activity inhibitor BIBR1532 reduced it more than 50%, as expected (Figure **7****).**

### Example 2.6. Aptamers do not affect terc transcript levels.

As the simplest theory of the aptamers mechanism of action is an indirect action through the modulation of endogenous *terc,* RNA levels of *terc* were measured by RT-qPCR. However, the results showed that transcript levels of *terc* in tested conditions were unaltered **(****Figure 8****)**.

### Example 2.7. Aptamers control the activity of myeloid gene promoters in a terc binding site-dependent manner.

It has been shown that *terc* regulates the expression of myeloid genes by binding to consensus sequences (Chu et al., 2011), called *terc* binding sites (*tercbs*), and by attracting the transcriptional machinery (Garcia-Castillo et al., unpublished). As these sequences are found in the *csf3b* promoter, it was cloned into a luciferase reporter for *in vivo* transcription activity assays. **Figure 9a** shows that while *CR4CR5* and *AA* aptamers were both able to increase wild type *csf3b* promoter activity at similar levels than full *terc,* that of the promoter lacking the *tercbs* did not change upon either *terc* or aptamers treatments **(****Figure 9b****).** As expected, *CR7* and *AAₘᵤₜ* aptamers were not able to increase the activity of either wild type or mutant *csf3b* promoters. Therefore, *tercbs* region was necessary for *csf3b* expression regulation by aptamers, as it happens with *terc.*

### Example 2.8. Aptamers directly bind to DNA.

Luciferase reporter experiments showed that aptamers can regulate the expression of master myeloid gene *csf3b* via interaction with *tercbs.* However, to confirm direct binding of aptamers to DNA, we performed an aptamers-DNA binding assay to evaluate whether aptamers were able to bind to putative *ter*cbs *in vitro.* A dsDNA biotinylated probe of *csf3b* upstream regulatory sequences (100 bp) containing *tercbs* was incubated with aptamers and *terc,* as a positive control, or luciferase mRNA (*luc*), as a negative control. In parallel, the same experiment was performed by using the *csf3b* promoter probe with mutant *tercbs* **(****Figure 10a**). Results showed that *terc* was able to bind *csf3b* probe with very hight affinity compared to control probe (*luc*) and that this interaction strongly decreased (3 times) when the *tercbs* mutant probe was used, as expected **(****Figure 10b**), demonstrating the relevance of the *tercbs* for *terc* binding to DNA and confirming the *in vivo* results. Similarly, *CR4CR5* aptamer robustly bound to wild type *csf3b* probe compare to *CR7* control, while *AA* and *AAₘᵤₜ* weakly interacted with the probe, all of them being largely *terc*bs-dependent **(****Figure 10c**). These results show that the *CR4CR5* and *AA* aptamers can directly bind the *tercbs* but with different affinity, and that the inability of the *AAₘᵤₜ* aptamer to enforce myelopoiesis is independent of its *tercbs* binding capacity.

### Example 2.9. Aptamers interact with RNA polymerase II.

The ability of aptamers to increase the number of neutrophils and monocytes through binding *tercbs* in the promoter of *csf3b* gene, led us to hypothesize that aptamers were acting as *terc.* Therefore, we next wanted to check whether they also interacted with RNA Pol II. An RNA pulldown procedure was performed using biotinylated aptamers incubated with protein extract from 5-day-old zebrafish larvae. Biotinylated *terc,* as positive control, and biotinylated GFP mRNA, as negative control, were also included. It was found that both *CR4CR5* and *AA* aptamers interacted with RNA Pol II with similar efficiency than *terc* **(****Figure 11****).** As expected, the negative control *CR7* and *AAₘᵤₜ* did not show statistically significant interaction with RNA Pol II.

### Example 2.10. Aptamers can rescue neutropenia in zebrafish disease models.

### Example 2.10.1. Aptamers rescue neutropenia in a DC model of terc deficiency.

*TERC* haploinsufficiency generate telomeropathies, such as DC, because telomerase is incapable of maintaining telomere length in tissues that need constant renewal, such as hematopoietic system (Mason and Bessler, 2011). Therefore, it was decided to test aptamers in heterozygous *terc* (*terc*^{+/-}) and homozygous (*terc*^{-/-}) zebrafish lines, which both show neutropenia at 11 hpf **(****Figure 12a****)** and telomere shortening (Hernández-Silva, personal communication). Notably, *CR4CR5* and *AA* aptamers were able to rescue neutropenia of both lines, as *terc* did **(****Figure 12a****).** This result is of high relevance from a therapeutic point of view, since it suggests the usefulness of aptamers to rescue neutropenia in DC patients with *TERC* haploinsufficiency and short telomeres.

### Example 2.10.2. Aptamers rescue neutropenia in a zebrafish model of poikiloderma with neutropenia.

It has been shown so far that aptamers can recover neutropenia in zebrafish models deficient in *terc.* However, in order to define the application capacity of these molecules, it was decided to test them in other human disease models.

There are few models of neutropenia in zebrafish. One of these models is a very rare autosomal recessive genodermatosis characterized by hypopigmentation or hyperpigmentation of different skin areas, skeletal defects and bone marrow alterations; called poikiloderma with neutropenia (PN) (Colombo et al., 2015, Patil et al., 2015). PN is caused by mutations in the U6 biogenesis 1 (*USB1*) gene, a 3'-5' exonuclease able to post-transcriptionally remove uridine and adenosine nucleosides from the 3' end of the spliceosomal U6 small nuclear RNA. Nearly half of PN patients develop myelodysplastic syndrome and acute myeloid leukemia in the second decade of life, following by bone marrow failure syndrome predisposing to cancer (Negri et al., 2015). Symptoms may be similar to DC, although the causes are different (Walne et al., 2016). The treatment of this disease is based on alleviating the symptoms, so, with respect to neutropenia, G-CSF is administered (Wang et al., 2017).

Previous zebrafish models of PN (Colombo et al., 2015, Patil et al., 2015) are based on the use of morpholinos against the *usbl* gene. However, in this work we have preferred to use CRISPR/Cas9 technology instead of morpholinos. To achieve this, genetic inactivation of *usbl* with CRISPR-Cas9 was performed in *lyz:dsRED,* instead of *mpx:GFP* zebrafish line, since it has been reported that *usbl* deficiency results in alteration of several genes involved in neutrophil differentiation and development, including *mpx* (Patil et al., 2015). We confirmed that the genetic inhibition of *usb1* resulted in decreased number of neutrophils in zebrafish **(****Figure 13a, 13b****).** In addition, *gcsf* mRNA could rescue neutropenia of this model **(****Figure 13a, 13b****),** as occurs in PN patients.

Once the model was validated, we used the aptamers to analyse their impact in neutropenia. Interestingly, both *CR4CR5* and *AA* aptamers were able to rescue neutropenia, as similar levels than *terc,* while the negative controls CR7 and *AAₘᵤₜ* failed to do so **(****Figure 14a****).** In addition, the same experiment was performed in the *mpx:GFP* transgenic line and the results were similar **(****Figure 14b****),** despite *usbl* deficiency may affect *mpx* expression. Therefore, *CR4CR5* and *AA* aptamers may be an effective treatment of human neutropenia that respond to G-CSF treatment.

### Example 2.11. Human aptamers.

*CR4CR5* and *AA* zebrafish aptamers have been shown to increase the number of neutrophils in both healthy and neutropenic fish models through the recruitment of RNA Pol II to myeloid gene promoters, such as *spi1* and *csf3b,* enhancing their expression. These results, together with the absence of apparent side effects in the model tested, suggest their usefulness to treat human diseases. Consequently, the same aptamers, but based on the human telomerase RNA structure, were synthetized and tested in human cell models of myelopoiesis, since the mechanism of *TERC* in myelopoiesis is highly conserved between human and zebrafish.

We decided to focus our research in *AA* and its mutant negative control *AAₘᵤₜ,* since *CR4CR5* aptamer worked similarly and the AA is smaller, so it has better therapeutic advantages and lower cost. The human aptamer *AA* (*hAA*) consisted of the 15 nucleotides (SEQ ID NO: 3) of the fork p6.1 of *CR4CR5* domain, had a similar structure to zebrafish *AA* aptamer and a 94% identity **(****Figure 15b****).** In addition, an aptamer control (h*AAₘᵤₜ*) (SEQ ID NO: 25) was designed with the mutation found in AA patients (G319A) (Carroll and Ly, 2009). As occurred in zebrafish, this mutation dramatically changes its structure **(****Figure 15c****).** As an additional control, a *hCR7* aptamer (46 nucleotides; SEQ ID NO: 26) was designed using as a template the ScaRNA domain, which is not involved in myelopoiesis and was a good control in zebrafish experiments **(****Figure 15a****).** All the human aptamers were chemically modified on the sugar ring, replacing the 2' position with an O-methyl group (OCH3), on the first two and last two bases of every aptamer to increase stability and prevent degradation by nucleases (reviewed by (Ni et al., 2017, Zhu and Chen, 2018, Kovacevic et al., 2018)). Furthermore, they were labelling at 3'end by Cy3 fluorescent dye to easily track them.

### Example 2.11.1 Effects of human aptamers in the promyelocytic HL60 cell line.

To test the effectiveness of the human aptamers, we chose the human neutrophil precursor cell line HL60, since it had already been used as a model in our laboratory to demonstrate that *TERC* was able to regulate the expression of *CSF2* (analogous to zebrafish *csf3b*) and *SPI1* (analogous to zebrafish *spi1b*) (Garcia-Castillo et al., unpublished). The first test was to check that aptamers could be taken up by the cells without the need of carriers. The results showed that this was the case **(****Figure 16a****).** As a result, only *hAA* aptamer was able to increase the transcript levels of *CSF2* **(****Figure 16c****),** but not those of *SPI1* **(****Figure 16d****)** or the balance between *SPI1* and *GATA1.* mRNA levels of *TERC* was unaffected, as expected **(****Figure 16b****).**

### Example 2.11.2 Effects of human aptamers in myeloid differentiation of iPS cells.

In order to evaluate the myeloproliferative potential of aptamers in humans, iPS cells were used. A healthy donor line (HD) was differentiated into hematopoietic cells through embryonic body (EB) formation, 3D cell aggregates that can differentiate into cells of all three germ layers. Within 15 days of differentiation, the cells were dissociated to perform a colony-forming unit (CFU) assay **(****Figure 17a****).** Consistent with our findings in zebrafish, while the control (C) and *hCR7* and *hAAₘᵤₜ* aptamer-treated cells were able to generate granulocytic-monocytic colonies (GM) with similar efficiency in CFU assays, cells treated with the *hAA* aptamer showed significantly increased percentage of GM-CFUs **(****Figure 17b****).** This result demonstrates that the *AA* aptamer is able to increase myelopoiesis in both zebrafish and human models.

## Claims

1. Affinity reagent specifically binding the TERC DNA-binding-site consisting of SEQ ID NO: 1 for use as a medicament.

2. Affinity reagent for use, according to claim 1, specifically binding the TERC DNA-binding-site consisting of SEQ ID NO: 1 at the regulatory regions of the genes CSF1, CSF2, CSF3 and SPI1.

3. Affinity reagent for use, according to any of the previous claims, in the treatment of neutropenia and/or the activation of myelopoiesis.

4. Affinity reagent for use, according to any of the previous claims, in the treatment of:
dyskeratosis congenita, poikiloderma with neutropenia, Charcot Marie tooth disease, non-immune chronic idiopathic neutropenia of adult, severe congenital neutropenia SCN8, SCN1, SCN3 or SCNX, anaemia x-linked with neutropenia and/or platelet abnormalities, cyclic neutropenia, Barth syndrome (BTHS), Shwachman-Diamond syndrome, Chédiak-Higashi syndrome, WHIM syndrome, and glycogen storage disease type Ib, hyper IgM syndrome, Hermansky-Pudlak syndrome (HPS), Griscelli syndrome (GS), P14 deficiency, Cohen syndrome, mobilization of hematopoietic stem cells or chemotherapy-induced neutropenia.

5. Affinity reagent for use, according to any of the previous claims, characterized it that it is an aptamer consisting of SEQ ID NO: 2 or SEQ ID NO: 3.

6. Aptamer consisting of SEQ ID NO: 2 or SEQ ID NO: 3.

7. Pharmaceutical composition comprising the aptamer of claim 6 and, optionally, pharmaceutically acceptable excipients or carriers.

8. *In vitro* method for screening affinity reagent candidates for the treatment of neutropenia and/or for the activation of myelopoiesis, which comprises determining whether the affinity reagent candidate binds the TERC DNA-binding-site consisting of SEQ ID NO: 1, wherein if it is determined that the affinity reagent candidate is able to bind the TERC DNA-binding-site consisting of SEQ ID NO: 1, the affinity reagent can be used for the treatment of neutropenia and/or for the activation of myelopoiesis.

9. *In vitro* method for screening affinity reagent candidates, according to claim 8, for the treatment of neutropenia and/or for the activation of myelopoiesis which comprises determining whether the affinity reagent candidate binds the TERC DNA-binding-site consisting of SEQ ID NO: 1 at the regulatory regions of the genes gcsfb and spi-1 like of zebrafish or CSF1, CSF2, CSF3 and SPI1 human genes, wherein if it is determined that the affinity reagent candidate is able to bind the TERC DNA-binding-site consisting of SEQ ID NO: 1 at the regulatory regions of the genes gcsfb and spi-1 like of zebrafish or CSF1, CSF2, CSF3 and SPI1 human genes, the affinity reagent can be used for the treatment of neutropenia and/or for the activation of myelopoiesis.

10. *In vitro* method for screening affinity reagent candidates, according to any of the claims 8 or 9, for the treatment of: dyskeratosis congenita, poikiloderma with neutropenia, Charcot Marie tooth disease, non-immune chronic idiopathic neutropenia of adult, severe congenital neutropenia SCN8, SCN1 or SCN3, x-linked severe congenital neutropenia, anaemia x-linked with neutropenia and/or platelet abnormalities, cyclic neutropenia, Barth syndrome (BTHS), Shwachman-Diamond syndrome, Chédiak-Higashi syndrome, WHIM syndrome, and glycogen storage disease type Ib, hyper IgM syndrome, Hermansky-Pudlak syndrome (HPS), Griscelli syndrome (GS), P14 deficiency, Cohen syndrome or chemotherapy-induced neutropenia, which comprises determining whether the affinity reagent candidate binds the TERC DNA-binding-site consisting of SEQ ID NO: 1 at the regulatory regions of gcsfb and spi-1 like genes of zebrafish or CSF1, CSF2, CSF3 and SPI1 human genes, wherein if it is determined that the affinity reagent candidate is able to bind the TERC DNA-binding-site consisting of SEQ ID NO: 1 at the regulatory regions of gcsfb and spi-1 like genes of zebrafish or CSF1, CSF2, CSF3 and SPI1 human genes, the affinity reagent can be used for the treatment of: dyskeratosis congenita, poikiloderma with neutropenia, Charcot Marie tooth disease, non-immune chronic idiopathic neutropenia of adult, severe congenital neutropenia SCN8, SCN1 or SCN3, x-linked severe congenital neutropenia, anaemia x-linked with neutropenia and/or platelet abnormalities, cyclic neutropenia, Barth syndrome (BTHS), Shwachman-Diamond syndrome, Chédiak-Higashi syndrome, WHIM syndrome, and glycogen storage disease type Ib, hyper IgM syndrome, Hermansky-Pudlak syndrome (HPS), Griscelli syndrome (GS), P14 deficiency, Cohen syndrome, mobilization of hematopoietic stem cells or chemotherapy-induced neutropenia.

11. *In vitro* method, according to any of the claims 8 to 10, for screening aptamer candidates.

12. *In vitro* method for generating affinity reagents specifically binding the TERC DNA-binding-site consisting of SEQ ID NO: 1 which comprises the use of the CR4/CR5 domain of TERC.

13. *In vitro* method for generating aptamers, according to claim 12, specifically binding the TERC DNA-binding-site consisting of SEQ ID NO: 1 which comprises the use of the CR4/CR5 domain of TERC.

14. *In vitro* use of CR4/CR5 domain of TERC for the generation of affinity reagents able to bind the TERC DNA-binding-site consisting of SEQ ID NO: 1.

15. *In vitro* use of CR4/CR5 domain of TERC, according to claim 14, for the generation of aptamers able to bind the TERC DNA-binding-site consisting of SEQ ID NO: 1.
